# EUROPÄISCHE PATENTSCHRIFT

(11) **EP 1 989 944 B1**
(45) Veröffentlichungstag und Bekanntmachung des Hinweises auf die Patenterteilung: **02.06.2010**
(21) Anmeldenummer: 08153790.4
(22) Anmeldetag: 31.03.2008
(51) Int. Cl.: A23L 1/226, C07C 233/58

(54) **Substituierte Cyclopropancarbonsäure(3-methyl-cyclohexyl)amide als Geschmacksstoffe**
Substituted cyclopropane carbolic acid(3-methyl-cyclohexyl)amides as taste substances
Acide cyclopropane carboxylique (3-méthyle-cyclohexyl)-amide substitué en tant qu'aromates

(30) Priorität: 08.05.2007 US 916589 P
(43) Veröffentlichungstag der Anmeldung: 12.11.2008
(73) Patentinhaber: Symrise GmbH & Co. KG, 37603 Holzminden (DE)
(72) Erfinder: Looft, Jan, 37603 Holzminden (DE); Vössing, Tobias, 37688 Beverungen (DE); Ley, Jakob, 37603 Holzminden (DE); Backes, Michael, 37603 Holzminden (DE); Blings, Maria, 37603 Holzminden (DE)
(74) Vertreter: Eisenführ, Speiser & Partner

(56) Entgegenhaltungen:
- US-A- 2 692 282
- US-A- 3 277 171
- US-A1- 2004 202 760
- US-A1- 2005 084 506

## Beschreibung

Die vorliegende Erfindung betrifft die Verwendung substituierter Cyclopropancarbonsäure(3-methyl-cyclohexyl)amide der Formel (I) (siehe unten) als Geschmacksstoffe. Die Verbindungen sind insbesondere zum Erzeugen, Modifizieren oder Verstärken eines Umami-Geschmacks geeignet. Die Erfindung betrifft des Weiteren bestimmte Zusammensetzungen und Halbfertigwaren, welche eine geschmacklich wirksame Menge der besagten Verbindungen umfassen sowie ein Verfahren zum Erzeugen, Modifizieren und/oder Verstärken bestimmter Geschmackseindrücke, insbesondere Umami. Schließlich betrifft die Erfindung auch neue Verbindungen, die besondere Geschmackseindrücke vermitteln.

Aromastoffe und Verbindungen mit außergewöhnlichen sensorischen Eigenschaften, die eine Amidgruppe tragen, sind seit langer Zeit bekannt. So haben viele bedeutende Kühlstoffe wie WS3, WS5 und WS23 eine Amidstruktur:

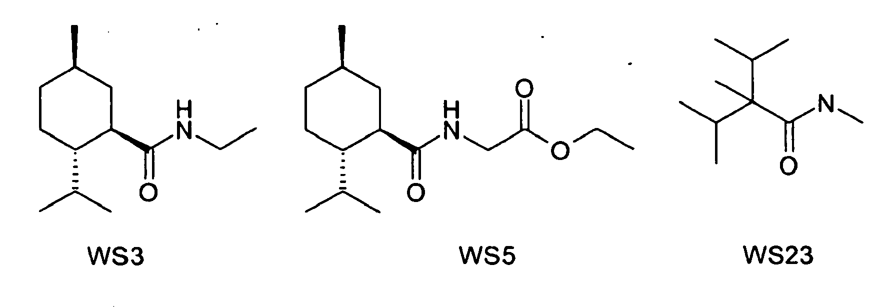

Ebenfalls zu den sensorisch bedeutsamen Amiden zählen die Scharfstoffe Capsaicin aus der Chilischote und das Piperin des weißen Pfeffers. Die natürlich vorkommenden Alkamide Pellitorin und Spilanthol zeigen neben einer speichelanregenden und kribbelnden Wirkung einen langanhaltenden und betäubenden Effekt im Mundraum:

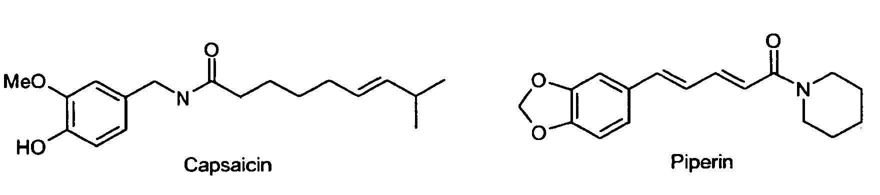

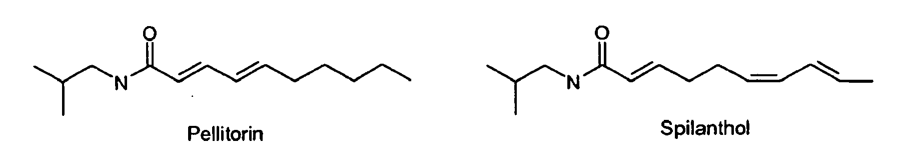

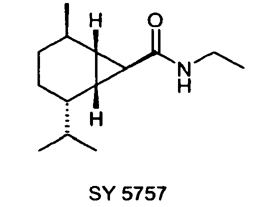

Angelehnt an die chemische Struktur des Spilanthols sind in den Veröffentlichungen US 2004/0202760 und US 2004/0202619 verschiedene Alkylidenamide vorgestellt worden, die ganz unterschiedliche sensorische Eindrücke wie Kribbeln, Betäuben, Bitterkeit, Mundfülle etc. umfassen. Für einige Verbindungen wie das N-Cyclopropyl-E2,Z6-nonadienamid, N-Ethyl-E2,Z6-dodecadienamid und N-Ethyl-E2,Z6-nonadienamid wird dabei eine MSG-ähnliche Wirkung (MSG = monosodium glutamate, Natriumglutamat) bzw. ein Umami-ähnlicher Eindruck angegeben.

In der Veröffentlichung US 2005/084506 wird eine Vielzahl angeblich geschmacksaktiver nicht-natürlicher Amide beschrieben.

In der US-Patentanmeldung 60/829,958 vom 18.10.2006 wurde - angelehnt an ein Mentholgrundgerüst - ein neuer künstlicher Geschmacksstoff vorgestellt, welcher besonders zum Erzeugen, Modifizieren und Verstärken eines UMAMI Geschmacks geeignet ist.

Mehrere Dokumente befassen sich mit im weitesten Sinne mit substituierten Cyclopropancarbonsäure(3-methyl-cyclohexyl)amiden und beschreiben deren pharmakologische Wirkungen.

In WO 2004/056745 werden Amine der Form 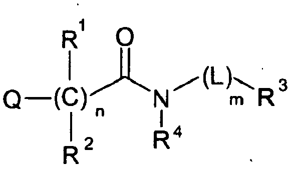 als 11-Beta Hydroxysteroid Dehydrogenase Inhibitoren beschrieben. Die allgemeinen Formeln in diesem Dokument sind jedoch so weit gefasst, dass eine so große Vielzahl von Verbindungen in sie hineininterpretiert werden könnte, dass als Offenbarung einzelner Verbindungen oder einer Gruppe von Verbindungen mit gemeinsamen Eigenschaften nur die konkreteren Angaben aufgefasst werden können, wie sie etwa ab Anspruch 3 im genannten Dokument offenbart werden. So fallen auch lange bekannte Verbindungen wie das oben erwähnte WS3 unter die obige allgemeine Formel (n,m = 0, R³ = C₁-C₈ Alkyl, R¹, R², R⁴ gleich Wasserstoff sowie Q gleich C₃ -C₈ Cycloalkyl mit bis zu drei Alkylsubstituenden). Der Geschmack der Verbindungen aus der WO 2004/056745 wird nicht beschrieben.

In WO 2005/020897 werden verschiedenste Derivate des Menthols als Trp-p8 Antagonisten beschrieben.

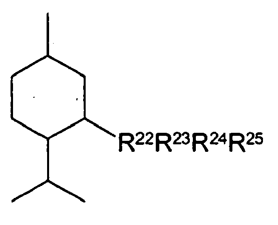

Allerdings ist auch hier die allgemeine Formel sehr weit gefasst (R²² u. A. AmidGruppe, R²³ u. A. aliphatische Gruppe mit bis zu bis 25 C sowie R²⁴ und R²⁵ = H), so dass WS3 wiederum mit eingeschlossen ist. Auch die folgende Verbindung ist nach der WO 2005/020897 möglich (Ref. No.: 2013):

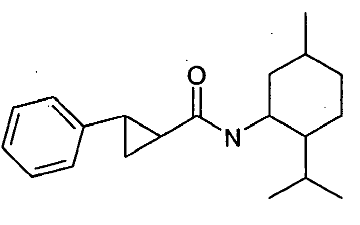

In diesem Dokument wird ebenfalls der Geschmack der beanspruchten Verbindungen nicht diskutiert.

EP 1632 483 beschäftigt sich mit heterocyclisch substituierten Carbonylderivaten als Liganden für den Dopamin D3 Rezeptor. Über den Geschmack ist auch in diesem Dokument nichts offenbart.

Des Weiteren ist eine Struktur bekannt, die nach CAPLUS Recherche kommerziell erhältlich ist [CAS 492426-03-6].

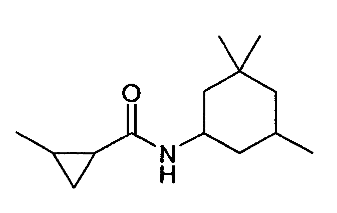

Über den Geschmack dieser Verbindung ist ebenfalls keine Aussage getroffen worden.

Weiterhin sind drei Dokumente bekannt, die über den Geschmack oder die Kühlwirkung von Cyclopropylcarbonsäurederivaten oder substituierten Cyclohexylamiden berichten.

In EP 1642 886 werden u. A. Cyclopropylcarbonsäurederivate folgender Struktur 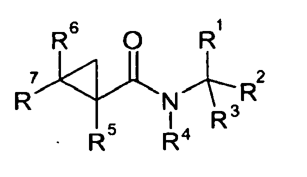 beschrieben, die zur Erzeugen, Modifizieren und/oder Verstärken mindestens einer der fünf Grundgeschmacksarten dienen. Allerdings liegt hier der Fokus auf ungesättigten Aminen. Allen Strukturen liegen zweifach alkylierten Amide zu Grunde und sofern Cyclohexylreste möglich sind, sind diese zwingend unsubtituiert.

In WO 2006/099762 wird die Verwendung verschiedenster substituierter Benzoecarbonsäureamide als Kühlstoffe beschrieben. Die auf Menthylamin basierende Verbindung 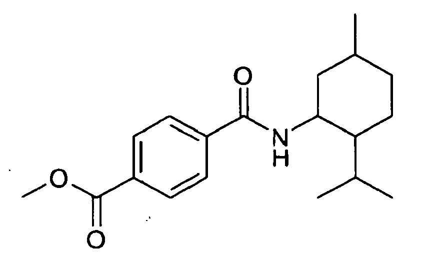 weist dabei die stärkste festgestellte Kühlwirkung auf.

In Dokument DE 2516610 von 1975 wird die Verwendung verschiedener Carbonsäurederivate (u. A. auch Amide) als Kühlwirkstoffe offenbart. Allerdings ist die allgemeine Formel sehr unspezifisch und in den Beispielen, die Cyclopropancarbonsäuren offenbaren, werden keine substituierten Cyclohexylamine verwendet.

Es besteht weiterhin Bedarf an der Auffindung neuer Geschmacks- und Aromastoffe, geschmacksaktiven Verbindungen oder an Verbindungen, die ein Aroma erzeugen, modifizieren oder verstärken können. Insbesondere besteht Bedarf an solchen Verbindungen, die den Geschmackseindruck "Umami" hervorrufen oder verstärken können.

Aufgabe der vorliegenden Erfindung war es daher, Mittel zur Verfügung zu stellen, mittels derer gewünschte Geschmacksnoten erzeugt, modifiziert oder verstärkt werden können.

Diese Aufgabe wird erfindungsgemäß gelöst durch die Verwendung einer Verbindung der Formel (I) 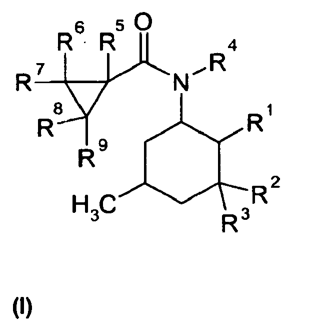 wobei gilt:
- R¹, R², R³: bedeuten jeweils unabhängig voneinander Wasserstoff oder einen Alkylrest von 1 bis 3 C-Atomen, insbesondere Methyl-, Ethyl-, n-Propyl- oder iso-Propyl-, wobei mindestens ein Rest ungleich H ist;
- R⁴: bedeutet Wasserstoff, einen Alkylrest von 1 bis 6 C-Atomen, insbesondere Methyl, Ethyl, n-Propyl, iso-Propyl, n-Butyl, iso-Butyl, sec.-Butyl, tert.-Butyl, n-Pentyl, 1-Methyl-butyl, 2-Methylbutyl, 3-Methyl-butyl, 1,2-Dimethyl-propyl, 1,1-Dimethyl-propyl, 2,2-Dimethyl-propyl, n-Hexyl, 1-Methyl-pentyl, 2-Methyl-pentyl, 3-Methyl-pentyl, 4-Methyl-pentyl, 1-Ethyl-butyl, 2-Ethyl-butyl, 1,1-Dimethyl-butyl, 1,2-Dimethyl-butyl, 1,3-Dimethyl-butyl, 2,2-Dimethyl-butyl, 2,3-Dimethyl-butyl, 3,3-Dimethyl-butyl, 1-Ethyl-1-methyl-propyl, 1-Ethyl-2-methyl-propyl, 1,1,2-Trimethyl-propyl oder 1,2,2-Trimethyl-propyl, oder einen Alkenylrest von 2 bis 6 C-Atomen, insbesondere 1-Propenyl, 2-Propenyl, 1-Methyl-1-propenyl, 2-Methyl-1-propenyl oder 2-Methyl-2-propenyl;
- R⁵, R⁶, R⁷, R⁸: bedeuten jeweils unabhängig voneinander Wasserstoff oder Methyl;
- R⁹: bedeutet Wasserstoff, einen Alkylrest mit 4 bis 12 C-Atomen insbesondere n-Butyl, iso-Butyl, sec.-Butyl, tert.-Butyl, n-Pentyl, 1-Methyl-butyl, 2-Methyl-butyl, 3-Methyl-butyl, 1,2-Dimethylpropyl, 1,1-Dimethyl-propyl, 2,2-Dimethyl-propyl, n-Hexyl, 1-Methyl-pentyl, 2-Methyl-pentyl, 3-Methyl-pentyl, 4-Methylpentyl, 1-Ethyl-butyl, 2-Ethyl-butyl, 1,1-Dimethyl-butyl, 1,2-Dimethyl-butyl, 1,3-Dimethyl-butyl, 2,2-Dimethyl-butyl, 2,3-Dimethyl-butyl, 3,3-Dimethyl-butyl, 1-Ethyl-1-methyl-propyl, 1-Ethyl-2-methyl-propyl, 1,1,2-Trimethyl-propyl, 1,2,2-Trimethylpropyl, n-Heptyl, n-Octyl, n-Nonyl, n-Decyl, n-Undecyl oder n-Dodecyl oder einen Alkenylrest mit 4 bis 12 C-Atomen, insbesondere 1-Methyl-1-propenyl, 2-Methyl-1-propenyl oder 2-Methyl-2-propenyl
als Geschmacksstoff oder Geschmacksstoffmischung.

Bevorzugt sind erfindungsgemäße Verbindungen der Formel (I), wobei die Verbindung oder die Verbindungen der Formel (I) eine Verbindung ist oder Verbindungen sind, ausgewählt aus der Gruppe bestehend aus:
(1) (1 R,3S)-2,2-Dimethyl-3-(2-methyl-propenyl)-cyclopropancarbonsäure-((1 S,2S,5R)-2-isopropyl-5-methyl-cyclohexyl)-amid
(2) (1R,3S)-2,2-Dimethyl-3-(2-methyl-propenyl)-cyclopropancarbonsäure-((1R,2R,5S)-2-isopropyl-5-methyl-cyclohexyl)-amid
(3) (1S,3R)-2,2-Dimethyl-3-(2-methyl-propenyl)-cyclopropancarbonsäure-((1S,2S,5R)-2-isopropyl-5-methyl-cyclohexyl)-amid
(4) (1S,3R)-2,2-Dimethyl-3-(2-methyl-propenyl)-cyclopropancarbonsäure-((1R,2R,5S)-2-isopropyl-5-methyl-cyclohexyl)-amid
(5) (1S,3S)-2,2-Dimethyl-3-(2-methyl-propenyl)-cyclopropancarbonsäure-((1S,2S,5R)-2-isopropyl-5-methyl-cyclohexyl)-amid
(6) (1S,3S)-2,2-Dimethyl-3-(2-methyl-propenyl)-cyclopropancarbonsäure-((1R,2R,5S)-2-isopropyl-5-methyl-cyclohexyl)-amid
(7) (1R,3R)-2,2-Dimethyl-3-(2-methyl-propenyl)-cyclopropancarbonsäure-((1S,2S,5R)-2-isopropyl-5-methyl-cyclohexyl)-amid
(8) (1R,3R)-2,2-Dimethyl-3-(2-methyl-propenyl)-cyclopropancarbonsäure-((1R,2R,5S)-2-isopropyl-5-methyl-cyclohexyl)-amid

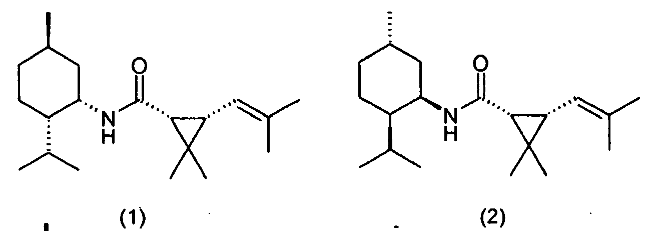

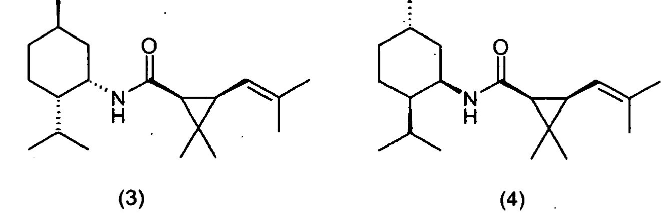

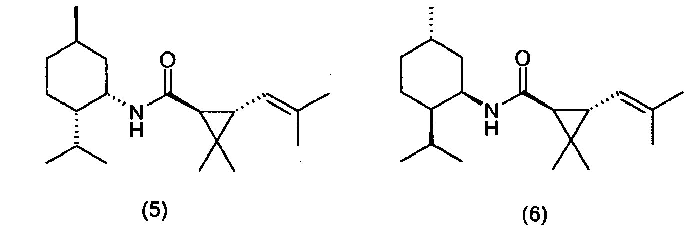

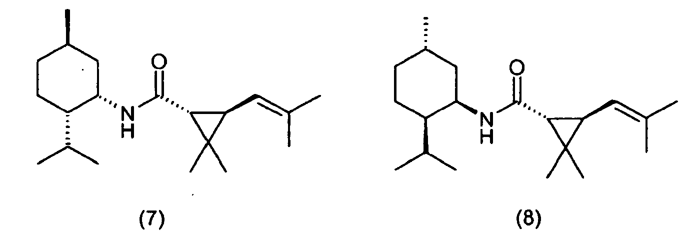

Bei der Suche nach solchen Verbindungen wurde überraschend festgestellt, dass - anders als durch den oben zitierten Stand der Technik nahegelegt - bestimmte substituierte Cyclopropancarbonsäure(3-methyl-cyclohexyl) amide der Formel (I) keine Kühlstoffe sind, sondern einen ausgeprägten Umami Charakter besitzen.

Die Bedeutung eines definierten Substitutionsmusters am Cyclohexangerüst wird weiterhin dadurch untermauert, dass das nicht erfindungsgemäße - in Anlehnung an EP 1642886 dargestellte - unsubstituierte Cyclohexanderivat 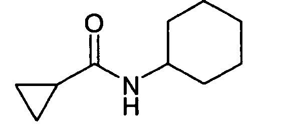 bei Verkostung in eigenen Untersuchungen als geschmacksneutral bewertet wurde. Auch in amerikanischem Rindfleischextrakt konnte insbesondere keine Erzeugung oder Verstärkung eines Umami Geschmackseindrucks bestätigt werden.

Gemäß einer Ausgestaltung der erfindungsgemäßen Verwendung ist es weiterhin bevorzugt, wenn die Verbindung oder die Verbindungen der Formel (I) als Reste R², R³, R⁴, R⁵, R⁶, R⁷,R⁸ jeweils Wasserstoff darstellen und somit eine Verbindung der Formel (II) 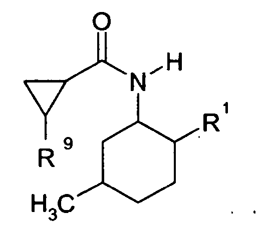 ist oder sind, wobei gilt:
- R¹: einen Alkylrest von 1 bis 3 C-Atomen, insbesondere Methyl-, Ethyl-, n-Propyl oder i-Propyl.
- R⁹: bedeutet Wasserstoff, einen Alkylrest mit 4 bis 12 C-Atomen insbesondere n-Butyl, iso-Butyl, sec.-Butyl, tert.-Butyl, n-Pentyl, 1-Methyl-butyl, 2-Methyl-butyl, 3-Methyl-butyl, 1,2-Dimethylpropyl, 1,1-Dimethyl-propyl, 2,2-Dimethyl-propyl, n-Hexyl, 1-Methyl-pentyl, 2-Methyl-pentyl, 3-Methyl-pentyl, 4-Methylpentyl, 1-Ethyl-butyl, 2-Ethyl-butyl, 1,1-Dimethyl-butyl, 1,2-Dimethyl-butyl, 1,3-Dimethyl-butyl, 2,2-Dimethyl-butyl, 2,3-Dimethyl-butyl, 3,3-Dimethyl-butyl, 1-Ethyl-1-methyl-propyl, 1-Ethyl-2-methyl-propyl, 1,1,2-Trimethyl-propyl, 1,2,2-Trimethylpropyl, n-Heptyl, n-Octyl, n-Nonyl, n-Decyl, n-Undecyl oder n-Dodecyl oder einen Alkenylrest mit 4 bis 12 C-Atomen, insbesondere Methyl-1-propenyl, 2-Methyl-1-propenyl oder 2-Methyl-2-propenyl.

Besonders bevorzugt sind erfindungsgemäße Verbindungen Formel (II) alle Mischungen sowie Einzelsubstanzen der folgenden Verbindungen:
(9) Cyclopropancarbonsäure((1 R,2S,5R)-2-isopropyl-5-methylcyclohexyl)-amid
(10) Cyclopropancarbonsäure((1S,2R,5S)-2-isopropyl-5-methylcyclohexyl)-amid
(11) Cyclopropancarbonsäure((1 S,2S,5R)-2-isopropyl-5-methylcyclohexyl)-amid
(12) Cyclopropancarbonsäure((1 R,2R,5S)-2-isopropyl-5-methylcyclohexyl)-amid
(13) Cyclopropancarbonsäure((1 R,2R,5R)-2-isopropyl-5-methylcyclohexyl)-amid
(14) Cyclopropancarbonsäure((1S,2S,5S)-2-isopropyl-5-methylcyclohexyl)-amid
(15) Cyclopropancarbonsäure((1 S,2R,5R)-2-isopropyl-5-methylcyclohexyl)-amid
(16) Cyclopropancarbonsäure((1 R,2S,5S)-2-isopropyl-5-methylcyclohexyl)-amid

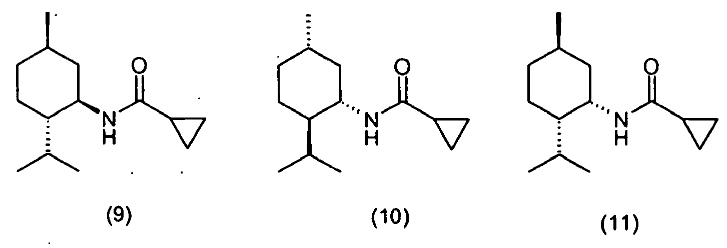

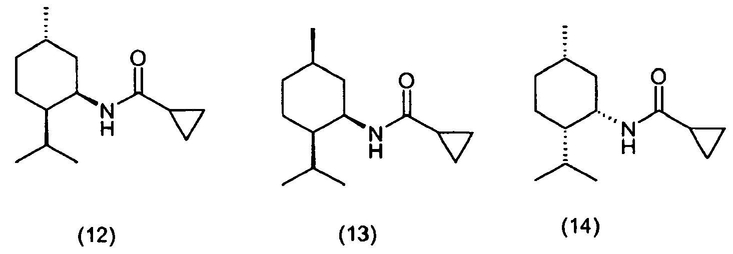

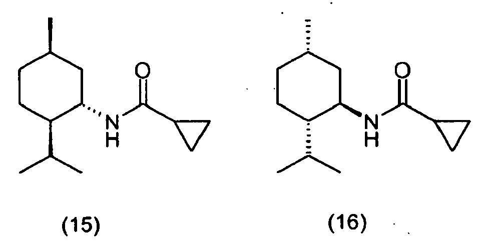

Besonders bevorzugt wegen Ihrer interessanten und starken Umami-Eigenschaften sind die Verbindungen der Formeln (11) und (12) oder eine Mischung umfassend oder bestehend aus den Verbindungen der Formeln (11) und (12).

Weiter besonders bevorzugt ist eine Mischung, welche aus folgenden Komponenten besteht oder diese enthält:
(a) eine Verbindung ausgewählt aus den Verbindungen der Formeln (11) und (12) oder eine Mischung bestehend aus den Verbindungen der Formeln (11) und (12)
   sowie
(b) eine Verbindung ausgewählt aus der Gruppe bestehend aus Verbindungen der Formeln (9), (10), (13), (14), (15) und (16) oder eine Mischung bestehend aus zwei oder mehreren Verbindungen ausgewählt aus der Gruppe bestehend aus Verbindungen der Formeln (9), (10), (13), (14), (15) und (16).

In der vorstehend definierten Mischung beträgt das Gewichtsverhältnis von (a) der Gesamtheit von Verbindungen der Formeln (11) und (12) zu (b) der Gesamtheit von Verbindungen der Formeln (9), (10), (13), (14), (15) und (16) vorzugsweise mindestens 60:40, weiter bevorzugt mindestens 90:10, insbesondere bevorzugt mindestens 95:5.

Gemäß einem weiteren Aspekt betrifft die vorliegende Erfindung auch Zusammensetzungen, insbesondere zum Verzehr geeignete Zusammensetzungen, umfassend oder bestehend aus
(i) einer geschmacklich wirksamen Menge einer oder mehrerer Verbindungen der Formeln (11) und (12) bzw. einer Mischung wie oben definiert bestehend aus (a) einer oder mehreren Verbindungen der Formeln (11) und (12) sowie (b) einer oder mehreren Verbindungen der Formeln (9), (10), (13), (14), (15), (16) sowie
(ii) einem oder mehreren weiteren zum Verzehr geeigneten Bestandteilen.

Dabei gilt hinsichtlich bevorzugter Verbindungen und Mischungen das oben Gesagte entsprechend.

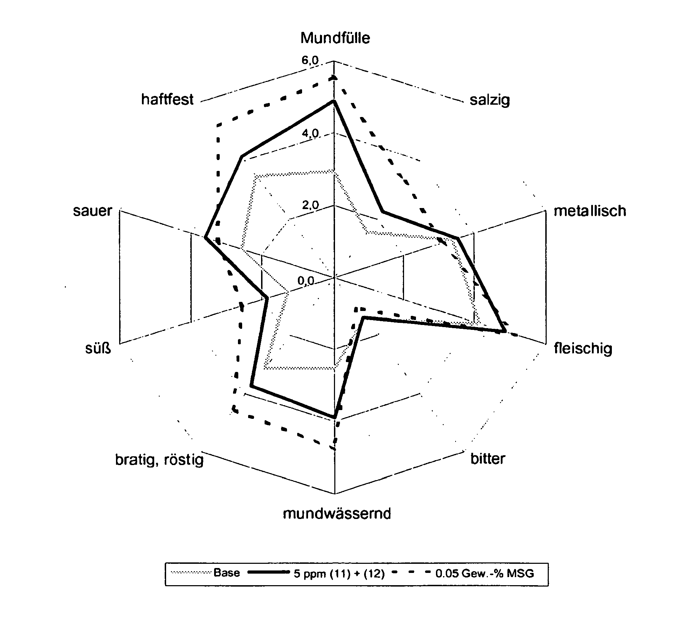

In dem als Figur 1 beigefügten Spiderdiagramm wird exemplarisch eine amerikanische Rindfleischbouillon als Base mit erstens einer solchen Base mit einem Zusatz von 5 ppm einer Mischung aus Cyclopropancarbonsäure((1S,2S,5R)-2-isopropyl-5-methyl-cyclohexyl)-amid (11) und Cyclopropancarbonsäure((1 R,2R,5S)-2-isopropyl-5-methyl-cyclohexyl)-amid (12) (Mischungsverhältnis 1 : 1) sowie zweitens mit einer solchen Base mit einem Zusatz von 0,05 Gew.-% MSG verglichen. Es wird deutlich, dass die oben genannten Verbindungen einen deutlichen Umami Geschmack besitzen, der im Profil dem Natriumglutamat sehr ähnlich ist.

In eigenen Untersuchungen hat sich gezeigt, dass die erfindungsgemäß zu verwendenden Verbindungen der Formel (I) oder (II) in stark Natriumglutamat-reduzierten oder Natriumglutamat-freien Nahrungsmitteln, so zum Beispiel in würzigen Nahrungsmitteln wie Tomatensuppe, Hühnersuppe, Knabbergebäck, Fertigpizza, Kartoffelchips und Popcorn einen Umami-Geschmack sowohl im Anfangsgeschmack (Impact) als auch in der längeranhaltenden Geschmackswahrnehmung besonders gut erzeugen, modifizieren und/oder verstärken können und daher das Geschmackserlebnis als angenehm empfunden wird, in vielen Fällen sogar als bevorzugt gegenüber Natriumglutamat.

Dementsprechend ist ein Teil der Erfindung auch die erfindungsgemäße Verwendung einer Verbindung oder einer Mischung von Verbindungen der Formel (I) oder (II) wie oben definiert zum Erzeugen, Modifizieren oder Verstärken eines Umami-Geschmackes.

Gemäß einem weiteren Aspekt betrifft die vorliegende Erfindung auch Zusammensetzungen, insbesondere zum Verzehr geeignete Zusammensetzungen, umfassend oder bestehend aus einer geschmacklich wirksamen Menge einer oder mehrerer Verbindungen der Formel (I) oder (II) sowie einem oder mehreren weiteren zum Verzehr geeigneten Bestandteil(en). Hinsichtlich der in der Zusammensetzung enthaltenen erfindungsgemäß verwendeten Verbindungen der Formel (I) oder (II) gilt das oben Gesagte entsprechend.

Die erfindungsgemäßen der Ernährung, der Mundpflege oder dem Genuss dienenden Zubereitungen (Zusammensetzungen) sind regelmäßig Produkte, die dazu bestimmt sind, in die menschliche Mundhöhle eingebracht zu werden, dort eine bestimmte Zeit zu verbleiben und anschließend entweder verzehrt (z.B. verzehrfertige Lebensmittel, siehe auch weiter unten) oder wieder aus der Mundhöhle entfernt zu werden (z.B. Kaugummis oder Zahnpasta). Zu diesen Produkten gehören dabei sämtliche Stoffe oder Erzeugnisse, die dazu bestimmt sind, in verarbeitetem, teilweise verarbeitetem oder unverarbeitetem Zustand vom Menschen aufgenommen zu werden. Hierzu zählen auch Stoffe, die Lebensmitteln bei ihrer Herstellung, Verarbeitung oder Bearbeitung zugesetzt werden und dazu vorgesehen sind, in die menschliche Mundhöhle eingebracht zu werden.

Im Rahmen des vorliegenden Textes werden unter einem "Lebensmittel" insbesondere Stoffe verstanden, die dazu bestimmt sind, in unverändertem, zubereitetem oder verarbeitetem Zustand vom Menschen geschluckt und dann verdaut zu werden; als Lebensmittel werden insoweit auch Umhüllungen, Überzüge oder sonstige Umschließungen verstanden, die dazu bestimmt sind, mitverschluckt zu werden, oder bei denen ein Verschlucken vorauszus ehen ist. Auch bestimmte Produkte, die üblicherweise wieder aus der Mundhöhle entfernt werden (z.B.

Kaugummis) sind im Rahmen des vorliegenden Textes als Lebensmittel zu verstehen, da bei ihnen nicht auszuschließen ist, dass sie zumindest teilweise geschluckt werden.

Unter einem verzehrfertigen Lebensmittel ist hierbei ein Lebensmittel zu verstehen, das hinsichtlich der für den Geschmack maßgeblichen Substanzen bereits vollständig zusammengesetzt ist. Unter den Begriff "verzehrfertiges Lebensmittel" fallen auch Getränke sowie feste oder halbfeste verzehrfertige Lebensmittel. Als Beispiele seien genannt Tiefkühlprodukte, die vor dem Verzehr aufgetaut und auf Verzehrtemperatur erwärmt werden müssen. Auch Produkte wie Joghurt oder Eiscreme aber auch Kaugummis oder Hartkaramellen zählen zu den verzehrfertigen Lebensmitteln.

Unter einem Mundpflegeprodukt (auch Mundhygieneprodukt oder mundhygienische Zubereitung genannt) im Sinne der Erfindung wird eine der dem Fachmann geläufigen Formulierungen zur Reinigung und Pflege der Mundhöhle und des Rachenraumes sowie zur Erfrischung des Atems verstanden. Hierbei ist die Pflege der Zähne und des Zahnfleisches ausdrücklich eingeschlossen. Darreichungsformen gebräuchlicher mundhygienischer Formulierungen sind insbesondere Cremes, Gele, Pasten, Schäume, Emulsionen, Suspensionen, Areosole, Sprays,wie auch Kapseln, Granulate, Pastillen, Tabletten, Bonbons oder Kaugummis, ohne dass diese Aufzählung für die Zwecke dieser Erfindung limitierend verstanden werden soll.

Bevorzugte Mundpflegeprodukte (Mundhygieneprodukt) sind insbesondere solche in Form von Zahnpasta, Zahncreme, Zahngel, Zahnpulver, Zahnputzflüssigkeit, Zahnputzschaum, Mundwasser, Zahncreme und Mundwasser als 2-in-1 Produkt, Lutschbonbon, Mundspray, Zahnseide, oder Zahnpflegekaugummi.

Kaugummis umfassen im allgemeinen eine Kaugummibase, d.h. eine beim Kauen plastisch werdende Kaumasse, Zucker verschiedener Arten, Zuckeraustauschstoffe, andere süß schmeckende Stoffe, Zuckeralkohole (insbesondere Sorbitol, Xylitol, Mannitol), Kühlwirkstoffe, Geschmackskorrigenzien für unangenehme Geschmackseindrücke, weitere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Feuchthaltemittel, Verdicker, Emulgatoren, Stabilisatoren, Geruchskorrigentien und Aromen (z.B.: Eycalyptus-Menthol, Kirsche, Erdbeere, Grapefruit, Vanille, Banane, Citrus, Pfirsich, schwarze Johannisbeere, Tropenfrüchte, Ingwer, Kaffee, Zimt, Kombinationen (der genannten Aromen) mit Mintaromen sowie Spearmint und Pfefferminz alleine). Besonders interessant ist auch die Kombination der Aromen mit weiteren Stoffen, die kühlende, wärmende und/oder mundwässerndernde Eigenschaften aufweisen.

Im Stand der Technik sind zahlreiche unterschiedliche Kaugummibasen bekannt, wobei zwischen sogenannten "chewing gum" - oder um "bubble gum" - Basen zu unterscheiden ist, wobei letztere weicher sind, damit sich damit auch Kaugummiblasen bilden lassen. Gängige Kaugummibasen umfassen neben traditionell eingesetzten natürlichen Harzen oder dem Naturlatex Chicle heute zumeist Elastomere wie Polyvinylacetate (PVA), Polyethylene, (nieder- oder mittelmolekulare) Polyisobutene (PIB), Polybutadiene, Isobuten-Isopren Copolymere (Butyl Rubber), Polyvinylethylether (PVE), Polyvinylbutylether, Copolymere von Vinylestern und Vinylethern, Styrol-Butadien-Copolymere (Styrol-Butadien-Rubber, SBR) oder Vinyl-Elastomere, z.B. auf Basis Vinylacetat/Vinyllaurat, Vinylacetat/Vinylstaerat oder Ethylen/Vinylacetat, sowie Mischungen der genannten Elastomere, wie beispielsweise in EP 0 242 325, US 4,518,615, US 5,093,136, US 5,266,336 US 5,601,858 oder US 6,986,709 beschrieben. Daneben umfassen Kaugummibasen weitere Bestandteile wie beispielsweise (mineralische) Füllstoffe, Weichmacher, Emulgatoren, Antioxidantien, Wachse, Fette oder fette Öle, wie beispielsweise gehärtete (hydrierte) pflanzliche oder tierische Fette, Mono-, Di- oder Triglyceride. Geeignete (mineralische) Füllstoffe sind beispielsweise Calciumcarbonat, Titandioxid, Siliciumdioxid, Talkum, Aluminiumoxid, Dicalciumphosphat, Tricalciumphosphat, Magnesiumhydroxid und deren Mischungen. Geeignete Weichmacher bzw. Mittel zur Verhinderung des Verklebens (detackifier) sind beispielsweise Lanolin, Stearinsäure, Natriumstearat, Ethylacetat, Diacetin (Gylcerindiacetat), Triacetin (Gylcerintriacetat), Triethylcitrat. Geeignete Wachse sind beispielsweise Paraffin Wachse, Candelilla Wachs, Carnauba Wachs, mikrokristalline Wachse und Polyethylen Wachse. Geeignete Emulgatoren sind beispielsweise Phosphatide wie Lecithin, Mono- und Diglyceride von Fettsäuren, z.B. Glycerin-monostearat.

Eine Reihe von erfindungsgemäßen Zusammensetzungen ist bevorzugt. Insbesondere bevorzugt ist eine (vorzugsweise sprühgetrocknete) Zusammensetzung, die neben der einen oder den mehreren erfindungsgemäß verwendeten Verbindungen der Formel (I) oder (II) einen oder mehrere zum Verzehr geeignete feste Trägerstoffe umfasst. Bevorzugte Zusammensetzungen bestehen aus der oder den erfindungsgemäß verwendeten Verbindungen der Formel (I) oder (II) sowie dem oder den Trägerstoffen.

Vorteilhafte Trägerstoffe in diesen bevorzugten erfindungsgemäßen (vorzugsweise sprühgetrockneten) Zusammensetzungen sind Siliciumdioxid (Kieselsäure, Kieselgel), Kohlenhydrate und/oder Kohlenhydratpolymere (Polysaccharide), Cyclodextrine, Stärken, abgebaute Stärken (Stärkehydrolysate), chemisch oder physikalisch modifizierte Stärken, modifizierte Cellulosen, Gummi Arabicum, Ghatti-Gummi, Traganth, Karaya, Carrageenan, Guarkernmehl, Johannisbrotkernmehl, Alginate, Pektin, Inulin oder Xanthan Gum zu nennen. Bevorzugte Stärkehydrolysate sind Maltodextrine und Dextrine.

Bevorzugte Trägerstoffe sind Siliciumdioxid, Gummi Arabicum und Maltodextrine, wobei hier wiederum Maltodextrine mit DE-Werten im Bereich 5 bis 20 bevorzugt sind. Es ist dabei unerheblich, welche Pflanze ursprünglich die Stärke zur Herstellung der Stärkehydrolysate geliefert hat. Geeignet und leicht verfügbar sind Mais-basierende Stärken sowie Stärken aus Tapioka, Reis, Weizen oder Kartoffeln. Die Trägerstoffe können dabei auch als Fließhilfsmittel fungieren, wie beispielsweise Siliciumdioxid.

Die erfindungsgemäßen Zusammensetzungen, die neben der oder den erfindungsgemäß verwendeten Verbindungen der Formel (I) oder (II) noch einen oder mehrere feste Trägerstoffe umfassen, können beispielsweise durch mechanische Mischvorgänge, wobei gleichzeitig auch eine Zerkleinerung der Partikel der vorliegende Partikel erfolgen kann, oder mittels Sprühtrocknung hergestellt werden. Bevorzugt sind erfindungsgemäße Zusammensetzungen, die feste Trägerstoffe umfassen und mittels Sprühtrocknung hergestellt sind; hinsichtlich der Sprühtrocknung wird verwiesen auf US 3,159,585, US 3,971,852, US 4,532,145 oder US 5,124,162.

Bevorzugte erfindungsgemäße, Trägerstoffe umfassende Zusammensetzungen, die mittels Sprühtrocknung hergestellt wurden, besitzen eine mittlere Partikelgrö-ße im Bereich von 30 - 300 µm und eine Restfeuchte von kleiner oder gleich 5 Gew.-%.

Das Gewichtsverhältnis der Gesamtmasse der erfindungsgemäß verwendeten Verbindungen der Formel (I) oder (II) zu dem oder den zur Ernährung geeigneten festen Trägerstoffen liegt vorzugsweise im Bereich von 1 : 10 bis 1 : 100000, bevorzugt im Bereich von 1 : 100 bis 1 : 20000, besonders bevorzugt im Bereich von 1 : 1000 bis 1 : 5000, bezogen auf die Trockenmasse der Zusammensetzung.

Die Summe der Bestandteile von (i) erfindungsgemäß zu verwendenden Verbindungen der Formel (I) oder (II) und (ii) dem bzw. den Trägerstoffen in der Zusammensetzung liegt vorzugsweise im Bereich von 70 bis 100 Gew.-%, bevorzugt im Bereich von 85 bis 100 Gew.-%.

Die Erfindung betrifft auch eine (vorzugsweise sprühgetrocknete) Zusammensetzung, die neben (i) einer oder mehreren erfindungsgemäß zu verwendenden Verbindungen der Formel (I) oder (II) sowie (ii) festen Trägerstoffen zusätzlich (iii) eine oder mehrere Aromakompositionen umfasst bzw. aus den genannten Komponenten besteht.

Eine solche Aromakomposition im Sinne der vorliegenden Erfindung umfasst mindestens einen flüchtigen Aromastoff (nicht gemeint sind hierbei allerdings Verbindungen der Formel (I) oder (II)). Der flüchtige Aromastoff ist dabei vorzugsweise eine sensorisch wirksame Komponente mit einem Dampfdruck von größer oder gleich 0,01 Pa bei 25°C, vorzugsweise einem Dampfdruck von größer oder gleich 0,025 Pa bei 25°C. Ein Großteil der flüchtigen Aromastoffe weist einen Dampfdruck von größer oder gleich 1 Pa bei 25°C auf, diese Aromastoffe werden für die Verwendung in erfindungsgemäßen Zusammensetzungen als bevorzugt angesehen.

Beispiele für Aromastoffe, die Bestandteil der Aromakomposition sein können, finden sich z.B. in K. Bauer, D. Garbe und H. Surburg, Common Fragrance and Flavor Materials, 4th. Ed., Wiley-VCH, Weinheim 2001. Es seien beispielsweise genannt: organische Säuren (gesättigt und ungesättigt) wie z.B. Buttersäure, Essigsäure, Methylbuttersäure, Capronsäure; Alkohole (gesättigt und ungesättigt) wie z.B. Ethanol, Propylenglykol, Octenol, cis-3-Hexenol, Benzylalkohol; Sulfide und Disulfide wie z.B. Dimethylsulfid, Difurfuryldisulfid, Methylthiopropanal; Thiole wie z.B. Methylfuranthiol; Pyrazine und Pyrroline wie z.B. Methylpyrazin, Acetylpyrazin, 2-Propionylpyrrolin, 2-Acetylpyrrolin.

Die Aromakomposition können auch in Form von Reaktionsaromen (Maillard-Produkte) und/oder Extrakten bzw. ätherischen Ölen von Pflanzen oder Pflanzenteilen bzw. Fraktionen davon eingesetzt werden.

Eine weitere bevorzugte, zum Verzehr geeignete erfindungsgemäße Zusammensetzung, welche eine oder mehrere erfindungsgemäß verwendete Verbindungen der Formel (I) oder (II) umfasst, ist eine Wasser-in-Öl (W/O) - Emulsion. Neben der oder den erfindungsgemäß verwendeten Verbindungen der Formel (I) oder (II) umfasst eine solche Emulsion Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung.

Vorzugsweise umfasst eine solche erfindungsgemäße Zusammensetzung (W/O - Emulsion)
- 0,01 bis 0,1 Gew.% einer oder mehrerer erfindungsgemäß verwendete Verbindungen der Formel (I) oder (II),
- 5 bis 30 Gew.-%, bevorzugt 8 bis 25 Gew.-% Wasser,
- 50 bis 90 Gew.-%, bevorzugt 60 bis 80 Gew.-% einer Ölphase,
- 0,1 bis 5 Gew.-% eines verzehrbaren W/O-Emulgators sowie
- gegebenenfalls ein oder mehrere Antioxidantien und gegebenenfalls ein oder mehrere Stoffe zur Verstärkung einer antioxidativen Wirkung.

Besonders bevorzugt besteht eine solche erfindungsgemäße W/O-Emulsion aus den genannten Bestandteilen in den genannten Mengen.

Die Ölphase einer solchen erfindungsgemäßen W/O-Emulssion umfasst ein (oder besteht aus einem) vorzugsweise fettes Öl und/oder eine Aromakomposition. Bevorzugt sind Ölphasen umfassend oder bestehend aus einem fetten Öl und einer Aromakomposition.

Als fette Öle eignen sich beispielsweise Speiseöle, insbesondere Pflanzenöle. Geeignete fette Öle sind beispielsweise Borretschöl, Distelöl, Erdnussöl, Haselnussöl, Kokosöl, Kürbiskernöl, Leinöl, Maiskeimöl, Makadamianussöl, Mandelöl, Olivenöl, Palmkernöl, Pekannussöl, Pistazienkernöl, Rapsöl, Reiskeimöl, Sesamöl, Sojaöl, Sonnenblumenöl, Walnussöl oder Weizenkeimöl, oder daraus erhältliche Fraktionen. Es können auch flüssige Neutralester basierend auf mittelkettigen Fettsäuren und Glycerin verwendet werden, wie beispielsweise Miglyole (z.B. Miglyol 810, Miglyol 812). Bevorzugt sind Sonnenblumenöl, Palmkernöl und Rapsöl. Ferner bevorzugt werden fraktionierte Kokosöle verwendet, die hauptsächlich Fettsäurereste mit 6 bis 8 C-Atomen aufweisen. Diese zeichnen sich durch ihre Geschmacksneutralität sowie durch ihre gute Oxidationsstabilität aus.

Vorzugsweise wird der verzehrbare W/O-Emulgator gewählt aus der Gruppe bestehend aus Lecithin (E 322), Mono- und Diglyceride von Speisefettsäuren (E 471), Essigsäuremonoglyceride (E 472a), Milchsäuremonoglyceride (E 472b), Citronensäuremonoglyceride (E 472c), Weinsäuremonoglyceride (E 472d), Diacetylweinsäuremonoglyceride (E 472e), Sorbitanmonostearat (E 491).

Geeignete Antioxidantien und Stoffe, die die antioxidative Wirkung verstärken können sind die natürlich vorkommenden Tocopherole und deren Derivate, Tocotrienole, Flavonoide, Ascorbinsäure und ihre Salze, alpha-Hydroxysäuren (z.B. Citronensäure, Milchsäure, Äpfelsäure, Weinsäure) und deren Na-, K- und Ca-Salze, aus Pflanzen isolierte Inhaltsstoffe, Extrakte bzw. Fraktionen davon z.B. aus Tee, Grüntee, Algen, Traubenkernen, Weizenkeimen, Rosmarin, Oregano, Flavonoide, Quercetin, phenolische Benzylamine. Weiterhin sind als Antioxidantien Propylgallat, Octylgallat, Dodecylgallat, Butylhydroxyanisol (BHA), Butylhydroxytoluol (BHT), Lecithine, Mono- und Diglyceride von Speisefettsäuren verestert mit Citronensäure, Orthophosphate und Na-, K- und Ca-Salze der Monophosphorsäure und Ascorbylpalmitat geeignet.

Die erfindungsgemäßen W/O-Emulsionen eignen sich besonders zum Aufbringen auf Lebensmitteloberflächen, wobei die Lebensmittel vorzugsweise einen Wassergehalt von höchstens 10 Gew.%, bevorzugt von höchstens 5 Gew.-% aufweisen. In einer bevorzugten Ausführungsform weist die erfindungsgemäße W/O - Emulsion bei Aufbringungstemperatur eine ausreichend niedrige Viskosität auf, so dass ein Aufbringen der W/O - Emulsion mittels Sprühen möglich ist. Bevorzugte Lebensmittel, auf deren Oberflächen eine erfindungsgemäße W/O - Emulsion aufgebracht werden kann sind beispielsweise Cracker, Chips (z.B. auf Basis von Kartoffeln, Mais, Getreide oder Brot), extrudierte Knabber (Snack) Artikel (z.B. Flips) oder Laugengebäck (z.B. Salzstangen). Erfindungsgemäße W/O - Emulsionen werden regelmäßig in einer Menge von 0,5 bis 6 Gew.-% auf die Lebensmitteloberflächen aufgebracht, bezogen auf das Gesamtgewicht des Lebensmittels.

Wie bereits erwähnt, betrifft ein Aspekt der vorliegenden Erfindung die Verwendung einer Verbindung der obigen Formel (I) oder (II) zum Erzeugen, Modifizieren oder Verstärken eines Umami-Geschmacks.

Vorzugsweise werden die erfindungsgemäß verwendeten Verbindungen der Formel (I) oder (II) (in geschmacklich wirksamer Menge) oder die erfindungsgemäßen Zusammensetzungen in der Ernährung oder dem Genuss dienenden (i) gebrauchs- oder verzehrfertigen Zubereitungen oder (ii) Halbfertigwaren eingesetzt, insbesondere in der Ernährung oder dem Genuss dienenden Natriumglutamat-reduzierten oder -freien Zubereitungen.

Der Begriff "Natriumglutamat-reduziert" bedeutet dabei, dass die erfindungsgemäße Zubereitung oder Halbfertigware deutlich weniger Natriumglutamat enthält, als in der üblichen Zubereitung oder Halbfertigware enthalten ist; der Natriumglutamatgehalt liegt dabei um 5 bis <100 Gew.-%, bevorzugt 10 bis 50 Gew.-%, besonders bevorzugt 20 bis 50 Gew.-% unter dem Natriumglutamatgehalt der üblichen Zubereitung. Sofern neben einer oder mehreren erfindungsgemäß verwendeten Verbindungen der Formel (I) oder (II) auch Natriumglutamat in einer erfindungsgemäßen Zubereitung oder Halbfertigware vorliegt, liegt das Gewichtsverhältnis der Gesamtmenge an Verbindungen der Formel (I) oder (II) zu Natriumglutamat vorzugsweise im Bereich von 1 : 1 bis 1 : 200.

Erfindungsgemäße der Ernährung oder dem Genuss dienende gebrauchs- oder verzehrfertige Zubereitungen enthalten eine oder mehrere erfindungsgemäß verwendete Verbindungen der Formel (I) oder (II) vorzugsweise in einer Menge im Bereich von 0,01 ppm bis 100 ppm, bevorzugt im Bereich von 0,1 ppm bis 50 ppm, insbesondere bevorzugt im Bereich von 1 ppm bis 30 ppm bezogen auf das Gesamtgewicht der gebrauchs- oder verzehrfertigen Zubereitung.

Erfindungsgemäße, der Ernährung oder dem Genuss dienende Halbfertigwaren enthalten eine oder mehrere erfindungsgemäß verwendete Verbindungen der Formel (I) oder (II) vorzugsweise in einer Menge im Bereich von 10 ppm bis 800 ppm, bevorzugt im Bereich von 25 ppm bis 750 ppm, insbesondere bevorzugt im Bereich von 50 ppm bis 700 ppm, bezogen auf das Gesamtgewicht der Halbfertigware.

Besonders relevant sind erfindungsgemäße Natriumglutamat-reduzierte Zubereitungen, die Natriumglutamat umfassen, wobei die Menge des Natriumglutamats nicht ausreicht, um in einer Vergleichszubereitung, die keine erfindungsgemäße Mischung umfasst, aber ansonsten identisch zusammengesetzt ist (normale Natriumglutamat-reduzierte Zubereitung), als befriedigender Umami-Geschmack wahrgenommen zu werden, und die Menge der erfindungsgemäßen Mischung ausreicht, um einen befriedigenden Umami-Geschmackseindruck zu erreichen.

Der Ernährung oder dem Genuss dienende Zubereitungen im Sinne der Erfindung sind insbesondere Backwaren (z.B. Brot, Trockenkekse, Kuchen, sonstiges Gebäck), Getränke (z.B. Gemüsesäfte, Gemüsesaftzubereitungen), Instantgetränke (z.B. Instant-Gemüsegetränke), Fleischprodukte (z.B. Schinken, Frischwurst- oder Rohwurstzubereitungen, gewürzte oder marinierte Frisch- oder Pökelfleischprodukte), gewürzte oder marinierte Fischprodukte (z.B. Surimi), Eier oder Eiprodukte (Trockenei, Eiweiß, Eigelb), Getreideprodukte (z.B. vorgegarte Fertigreis-Produkte, Reismehlprodukte, Hirse- und Sorghum-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Gemüsezubereitungen (z.B. Ketchup, Soßen, Trockengemüse, Tiefkühlgemüse, vorgegarte Gemüse, in Essig eingelegte Gemüse, Gemüsekonzentrate oder -pasten, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais-, Reis- oder Erdnussbasis), Produkte auf Fett- und Ölbasis oder Emulsionen derselben (z.B. Mayonnaise, Aufstrich, Remoulade, Dressings, Würzzubereitungen), sonstige Fertiggerichte und Suppen (z.B. Trockensuppen, Instant-Suppen, vorgegarte Suppen), Soßen (Instant-Soßen, Trockensoßen, Fertigsoßen), Gewürze oder Gewürzzubereitungen (z.B. Senfzubereitungen, Meerrettichzubereitungen), Würzmischungen sowie insbesondere Aufstreuwürzungen (englisch: Seasonings), die beispielsweise im Snackbereich Anwendung finden.

Besonders bevorzugt sind (Natriumglutamat-reduzierte) Natriumglutamat-haltige Halbfertigwaren oder der Ernährung oder dem Genuss dienende Zubereitungen, z.B. Backwaren (z.B. Brot, Trockenkekse, Kuchen, sonstiges Gebäck), Gemüsesaftzubereitungen, Fleischprodukte (z.B. Schinken, Frischwurst- oder Rohwurstzubereitungen, gewürzte oder marinierte Frisch- oder Pökelfleischprodukte), gewürzte oder marinierte Fischprodukte (z.B. Surimi), Eier oder Eiprodukte (Trockenei, Eiweiß, Eigelb), Getreideprodukte (z.B. vorgegarte Fertigreis-Produkte, rohe oder vorgegarte Nudeln und Pastaprodukte), Milchprodukte (z.B. Frischkäse, Weichkäse, Hartkäse, Milchgetränke, Molke, Butter, teilweise oder ganz hydrolysierte Milchprotein-haltige Produkte), Produkte aus Sojaprotein oder anderen Sojabohnen-Fraktionen (z.B. Sojamilch und daraus gefertigte Produkte, Sojalecithin-haltige Zubereitungen, fermentierte Produkte wie Tofu oder Tempe oder daraus gefertigte Produkte, Sojasoßen), Fischsoßen wie z.B. Anchoviso-ßen, Austernsoßen, Gemüsezubereitungen (z.B. Ketchup, Soßen, Trockengemüse, Tiefkühlgemüse, vorgegarte Gemüse, in Essig eingelegte Gemüse, eingekochte Gemüse, Kartoffelzubereitungen), Knabberartikel (z.B. gebackene oder frittierte Kartoffelchips oder Kartoffelteigprodukte, Brotteigprodukte, Extrudate auf Mais- oder Erdnussbasis), Produkte auf Fett- und Ölbasis oder Emulsionen derselben (z.B. Mayonnaise, Aufstrich, Remoulade, Dressings, Würzzubereitungen), Fertiggerichte, Suppen (z.B. Trockensuppen, Instant-Suppen, vorgegarte Suppen), Brühwürfel, Soßen (Instant-Soßen, Trockensoßen, Fertigsoßen), Gewürze, Würze, Würzmittel, Würzmischungen sowie insbesondere Aufstreuwürzungen (englisch: Seasonings), die beispielsweise im Snackbereich Anwendung finden.

Die Zubereitungen im Sinne der Erfindung können auch in Form von Kapseln, Tabletten (nichtüberzogene sowie überzogene Tabletten, z.B. magensaftresistente Überzüge), Dragees, Granulaten, Pellets, Feststoffmischungen, Dispersionen in flüssigen Phasen, als Emulsionen, als Pulver, als Lösungen, als Pasten oder als andere schluck- oder kaubare Zubereitungen vorliegen, z. B. als Nahrungsergänzungsmittel.

Die erfindungsgemäßen, Halbfertigwaren dienen in der Regel zur Herstellung von der Ernährung oder dem Genuss dienenden gebrauchs- oder verzehrfertigen Zubereitungen.

Insbesondere können erfindungsgemäße, der Ernährung oder dem Genuss dienende Halbfertigwaren zur additiven Verstärkung des Umami-Geschmacks von Natriumglutamat-reduzierten Nahrungs- und Genussmitteln und auch direkt als Würzmittel für die industrielle oder nicht-industrielle Zubereitung von Nahrungs- und/oder Genussmitteln dienen.

Erfindungsgemäße Halbfertigwaren enthalten bevorzugt eine Gesamtmenge von 10 ppm bis 800 ppm, bevorzugt 25 ppm bis 750 ppm, insbesondere 50 ppm bis 700 ppm, an erfindungsgemäß zu verwendenden Verbindungen der Formel (I) oder (II),
und/oder kein Natriumglutamat
und/oder einen Anteil von 0,00001 bis 10 Gew.-%, bevorzugt 0,0001 bis 5 Gew.-%, insbesondere 0,001 Gew.-% bis 2 Gew.-% an Natriumglutamat, (jeweils alternativ zu keinem Natriumglutamat),
und gegebenenfalls einen Anteil von 0,0001 Gew.-% bis 90 Gew.-%, bevorzugt 0,001 Gew.-% bis 30 Gew.-% einer Aromakomposition, jeweils bezogen auf das Gesamtgewicht der Halbfertigware.

Die erfindungsgemäßen Zubereitungen bzw. Halbfertigwaren werden vorzugsweise hergestellt, indem die erfindungsgemäß zu verwendenden Verbindungen der Formel (I) oder (II) in Mischungen aus Ethanol und gegebenenfalls demineralisiertem und/oder gereinigtem Wasser gelöst und gemischt werden; anschlie-ßend werden die Lösungen durch einen Trocknungsprozess, vorzugsweise einen Sprühtrocknungs-, Vakuumgefriertrockungs-, Umkehrosmose-, Eindampf- oder anderen Konzentrationsprozess oder eine Kombination der genannten Prozesse, in eine (zumindest nahezu) feste Zubereitung überführt. Dabei kann die Trocknung unter Zuhilfenahme von Trägerstoffen (z.B. Stärke, Stärkederivate, Maltodextrin, Kieselgel, siehe oben) oder Hilfsstoffen (z.B. Pflanzengummen, Stabilisierungsmittel), erfolgen. Vorzugsweise erfolgt die Trocknung mittels Sprühtrocknung oder Vakuumgefriertrocknung.

Bevorzugte erfindungsgemäße Zubereitungen bzw. Halbfertigwaren sind Würze, Würzmischungen, Würzmittel, Brühwürfel, Instant-Suppen, Instant-Soßen, vegetarische Fertiggerichte, fleischhaltige Fertiggerichte, Fischsoßen wie z.B. Anchovi-Soßen, Austernsoßen und Sojasoßen.

Gemäß einer weiteren bevorzugten Ausführungsform werden zur Herstellung erfindungsgemäßer Zubereitungen beziehungsweise Halbfertigwaren erfindungsgemäß zu verwendende Verbindungen der Formel (I) oder (II) sowie gegebenenfalls andere Bestandteile zunächst in Emulsionen, in Liposomen (z.B. ausgehend von Phosphatidylcholin) in Microsphären, in Nanosphären oder auch in Kapseln, Granulate oder Extrudate aus einer für Lebens- und Genussmittel geeigneten Matrix (z.B. aus Stärke, Stärkederivaten, Cellulose oder Cellulosederivaten wie Hydroxypropylcellulose, anderen Polysacchariden wie Alginat, natürlichen Fetten, natürlichen Wachsen wie Bienenwachs oder Carnaubawachs oder aus Proteinen wie Gelatine) eingearbeitet.

In einem weiteren bevorzugten Herstellungsverfahren werden die erfindungsgemäß zu verwendenden Verbindungen der Formel (I) oder (II) mit einem oder mehreren geeigneten Komplexbildnern, beispielsweise mit Cyclodextrinen oder Cyclodextrinderivaten, bevorzugt alpha- oder beta-Cyclodextrin, komplexiert und in dieser komplexierten Form eingesetzt.

Besonders bevorzugt sind erfindungsgemäße Zubereitungen, bei denen die Matrix so gewählt ist, dass die erfindungsgemäß zu verwendenden Verbindungen der Formel (I) oder (II) verzögert von der Matrix freigegeben werden, so dass man eine langanhaltende Wirkung erhält. Als Matrix können hier z.B. natürliche Fette, natürliche Wachse (z.B. Bienenwachs, Carnaubawachs) oder auch natürliche Ballaststoffe (Weizenfasern, Apfelfasern, Haferfasern, Orangenfasern) verwendet werden.

Weitere Bestandteile einer erfindungsgemäßen, der Ernährung oder dem Genuss dienenden verzehrfertigen Zubereitung bzw. Halbfertigware können übliche Grund-, Hilfs- und Zusatzstoffe für Nahrungs- oder Genussmittel sein, z.B. Wasser, Gemische frischer oder prozessierter, pflanzlicher oder tierischer Grund- oder Rohstoffe (z.B. rohes, gebratenes, getrocknetes, fermentiertes, geräuchertes und/oder gekochtes Fleisch, Knochen, Knorpel, Fisch, Gemüse, Kräuter, Nüsse, Gemüsesäfte oder -pasten oder deren Gemische), verdauliche oder nicht verdauliche Kohlenhydrate (z.B. Saccharose, Maltose, Fructose, Glucose, Dextrine, Amylose, Amylopektin, Inulin, Xylane, Cellulose, Tagatose), Zuckeralkohole (z.B. Sorbit, Erythritol), natürliche oder gehärtete Fette (z.B. Talg, Schmalz, Palmfett, Kokosfett, gehärtetes Pflanzenfett), Öle (z.B. Sonnenblumenöl, Erdnussöl, Maiskeimöl, Olivenöl, Fischöl, Sojaöl, Sesamöl), Fettsäuren oder deren Salze (z.B. Kaliumstearat), proteinogene oder nicht-proteinogene Aminosäuren und verwandte Verbindungen (z.B. γ-Aminobuttersäure, Taurin), Peptide (z.B. Glutathion), native oder prozessierte Proteine (z.B. Gelatine), Enzyme (z.B. Peptidasen), Nucleinsäuren, Nucleotide, Geschmackskorrigenzien für unangenehme Geschmackseindrücke, weitere Geschmacksmodulatoren für weitere, in der Regel nicht unangenehme Geschmackseindrücke, andere geschmacksmodulierende Stoffe (z.B. Inositolphosphat, Nucleotide wie Guanosinmonophosphat, Adenosinmonophosphat oder andere Stoffe wie Natriumglutamat oder 2-Phenoxypropionsäure), Emulgatoren (z.B. Lecithine, Diacylglycerole, Gummi arabicum), Stabilisatoren (z.B. Carrageenan, Alginat), Konservierungsstoffe (z.B. Benzoesäure und deren Salze, Sorbinsäure und deren Salze), Antioxidantien (z.B. Tocopherol, Ascorbinsäure), Chelatoren (z.B. Citronensäure), organische oder anorganische Säuerungsmittel (z.B. Essigsäure, Phosphorsäure), zusätzliche Bitterstoffe (z.B. Chinin, Coffein, Limonin, Amarogentin, Humolone, Lupolone, Catechine, Tannine), die enzymatische Bräunung verhindernde Stoffe (z.B. Sulfit, Ascorbinsäure), etherische Öle, Pflanzenextrakte, natürliche oder synthetische Farbstoffe oder Farbpigmente (z.B. Carotinoide, Flavonoide, Anthocyane, Chlorophyll und deren Derivate), Gewürze, trigeminal wirksame Stoffe oder Pflanzenextrakte, enthaltend solche trigeminal wirksamen Stoffe, synthetische, natürliche oder naturidentische Aromastoffe oder Riechstoffe sowie Geruchskorrigenzien.

Bevorzugt enthalten erfindungsgemäße Zusammensetzungen, Zubereitungen oder Halbfertigwaren eine Aromakomposition, um den Geschmack und/oder den Geruch abzurunden und zu verfeinern. Eine erfindungsgemäße Zusammensetzung, die als weitere Bestandteile einen festen Trägerstoff und eine Aromakomposition umfasst, wurde bereits weiter oben beschrieben. Geeignete Aromakompositionen enthalten z.B. synthetische, natürliche oder naturidentische Aroma-, Riech- und Geschmacksstoffe, Reaktionsaromen, Raucharomen oder andere aromagebende Zubereitungen (z.B. Protein[teil]hydrolysate, Grillaromen, Pflanzenextrakte, Gewürze, Gewürzzubereitungen, Gemüse und/oder Gemüsezubereitungen) sowie geeignete Hilfs- und Trägerstoffe. Insbesondere sind hier die nicht-erfindungsgemäßen Aromenkompositionen oder deren Bestandteile geeignet, die einen röstigen, fleischigen (insbesondere Huhn, Fisch, Meerestiere, Rind, Schwein, Lamm, Schaf, Ziege), gemüsigen (insbesondere Tomate, Zwiebel, Knoblauch, Sellerie, Lauch, Pilze, Auberginen, Seetang), einen würzigen (insbesondere schwarzer und weißer Pfeffer, Chili, Paprika, Kardamom, Muskat, Piment, Senf und Senf-Produkte), gebratenen, hefigen, gesottenen, fettigen, salzigen und/oder scharfen Aromaeindruck verursachen und somit den würzigen Eindruck verstärken können. In der Regel enthalten die Aromakompositionen mehr als einen der genannten Inhaltsstoffe.

In einer weiteren Ausgestaltung der vorliegenden Erfindung werden die erfindungsgemäß zu verwendenden Verbindungen der Formel (I) oder (II) in den erfindungsgemäßen Zusammensetzungen, Zubereitungen und Halbfertigwaren in Kombination mit zumindest einer (weiteren, nicht für sich erfindungsgemäßen) Substanz zum Maskieren oder Vermindern eines unangenehmen (bitteren, metallischen, kalkigen, sauren, adstringierenden) Geschmackseindrucks oder zur Verstärkung oder Erzeugung eines angenehmen Geschmackseindrucks (süß, salzig, Umami) verwendet. Auf diese Weise kann eine Verstärkung des Geschmacks, insbesondere des Umami-Geschmacks, erreicht werden. Diese weiteren Substanzen können aus der folgenden Liste ausgewählt werden, ohne die Erfindung damit einzuschränken: Mononatriumglutamat, Glutaminsäure, Nucleotide (z.B. Adenosin-5'-monophosphat, Cytidin-5'-monophosphat, Inosin-5'-monophosphat, Guanosin-5'-monophosphat) oder deren pharmazeutisch akzeptable Salze, Lactisole, Hydroxyflavanone (z.B. Eriodictyol, Homoeriodictyol oder deren Natriumsalze), insbesondere gemäß EP 1 258 200, Hydroxybenzoesäureamide (z. B. 2,4-Dihydroxybenzoesäurevanillylamid, 4-Hydroxybenzoesäurevanillylamid), Gemische von Molkeproteinen mit Lecithinen, Hefeextrakte, Pflanzenhydrolysate, pulverisierte Gemüse (z.B. Zwiebelpulver, Tomatenpulver), Pflanzenextrakte (z.B. von Liebstöckel oder von Pilzen wie Shiitake), Meeralgen und Mineralsalzmischungen.

Weitere vorteilhfate modulierende Aroma- und/oder Geschmackstoffe werden vorzugsweise ausgewählt aus der Gruppe bestehend aus 2,4-Dihydroxybenzoesäure; 3-Hydroxybenzoesäure; Natriumsalzen, vorzugsweise Natriumchlorid, Natriumlactat, Natriumcitrat, Natriumacetat, Natriumgluconoat; Hydroxybenzoesäureamiden, wie zum Beispiel 2,4-Dihydroxybenzoesäure-*N*-(4-hydroxy-3-methoxybenzyl)amid, 2,4,6-Trihydroxybenzoesäure-*N*-(4-hydroxy-3-methoxybenzyl)amid, 2-Hydroxy-benzoesäure-*N*-4-(hydroxy-3-methoxybenzyl)amid, 4-Hydroxybenzoesäure-N-(4-hydroxy-3-methoxybenzyl)-amid, 2,4-Dihydroxybenzoesäure-N-(4-hydroxy-3-methoxybenzyl)amid-Mono-natriumsalz, 2,4-Dihydroxybenzoesäure-N-2-(4-hydroxy-3-methoxyphenyl)-ethyl-amid, 2,4-Dihydroxybenzoesäure-N-(4-hydroxy-3-ethoxybenzyl)amid, 2,4-Dihydroxybenzoesäure-*N*-(3,4-dihydroxybenzyl)amid und 2-Hydroxy-5-methoxy-N-[2-(4-hydroxy-3-methoxyphenyl)ethyl]amid; (insbesondere solche wie beschrieben in WO 2006/024587, die bezüglich der darin offenbarten entsprechenden Verbindungen auf dem Wege der Verweisung Bestandteil dieser Anmeldung wird); Hydroxydeoxybenzoinen, wie zum Beispiel 2-(4-Hydroxy-3-methoxyphenyl)-1-(2,4,6-trihydroxyphenyl)ethanon, 1-(2,4-Dihydroxyphenyl)-2-(4-hydroxy-3-methoxyphenyl)ethanon, 1-(2-Hydroxy-4-methoxyphenyl)-2-(4-hydroxy-3-methoxyphenyl) ethanon) (insbesondere solche wie beschrieben in WO 2006/106023, die bezüglich der darin offenbarten entsprechenden Verbindungen auf dem Wege der Verweisung Bestandteil dieser Anmeldung wird); Hydroxyphenlaalkandionen, wie zum Beispiel Gingerdion-[2], Gingerdion-[3], Gingerdion-[4], Dehydrogingerdion-[2], Dehydrogingerdion-[3], Dehydrogingerdion-[4]) (insbesondere solche wie beschrieben in WO 2007/003527, die bezüglich der darin offenbarten entsprechenden Verbindungen auf dem Wege der Verweisung Bestandteil dieser Anmeldung wird); Diacetyltrimeren (insbesondere solche wie beschrieben in WO 2006/058893, die bezüglich der darin offenbarten entsprechenden Verbindungen auf dem Wege der Verweisung Bestandteil dieser Anmeldung wird); γ-Aminobuttersäuren (insbesondere solche wie beschrieben in WO 2005/096841, die bezüglich der darin offenbarten entsprechenden Verbindungen auf dem Wege der Verweisung Bestandteil dieser Anmeldung wird) und Divanillinen (insbesondere Divanillin wie beschrieben in WO 2004/078302, die bezüglich der darin offenbarten entsprechenden Verbindungen auf dem Wege der Verweisung Bestandteil dieser Anmeldung wird); Bicyclo[4.1.0]heptan-7-carbonsäureamide, insbesondere solche wie beschrieben in PCT/EP2007/061171 sowie den darauf basierenden Dokumenten (Symrise), die bezüglich der darin offenbarten entsprechenden Verbindungen auf dem Wege der Verweisung Bestandteil dieser Anmeldung wird.

In einer bevorzugten Ausgestaltung der vorliegenden Erfindung werden die erfindungsgemäß zu verwendenden Verbindungen der Formel (I) oder (II) in den erfindungsgemäßen Zusammensetzungen, Zubereitungen und Halbfertigwaren in Kombination mit zumindest einem süßverstärkenden Stoff eingesetzt, insbesondere mit einer oder mehreren Verbindungen gemäß WO 2007/014879 A1 oder WO 2007/107596 A1, speziell zusammen mit Hesperetin und/oder Phloretin. Hierdurch wird eine Verstärkung und eine Vertiefung sowie Abrundung des Geschmacksprofils erzielt, inbesondere in Zusammensetzungen, Zubereitungen und Halbfertigwaren mit würzigem und/oder salzigem Geschmack. Der Gesamtanteil an Hesperetin und/oder Phloretin in solchen Zusammensetzungen oder Zubereitungen liegt vorzugsweise im Bereich von 1 bis 400 ppm, bevorzugt im Bereich von 5 - 200 ppm, bezogen auf das Gesamtgewicht der Zusammensetzung oder Zubereitung.

Zusätzlich zu einem oder mehreren süßverstärkenden Stoffen können in den erfindungsgemäßen Zusammensetzungen, Zubereitungen und Halbfertigwaren vorzugsweise Geschmackstoffe enthalten sein, die einen trigeminalen Reiz (tingling, kribbeln, scharf, kühlend etc.) bewirken. So wurde bei der Kombination der erfindungsgemäß zu verwendenden Verbindungen der Formel (I) oder (II) mit Hesperetin und/oder Phloretin einerseits und cis- und/oder trans-Pellitorin (siehe WO 2004/000787 bzw. WO 2004/043906) andererseits ein weiter verbessertes und von Konsumenten bevorzugtes Geschmacksprofil erreicht. Der Gesamtanteil an cis- und/oder trans-Pellitorin in solchen Zusammensetzungen oder Zubereitungen liegt vorzugsweise im Bereich von 0,5 bis 500 ppm, bevorzugt im Bereich von 5 - 100 ppm, bezogen auf das Gesamtgewicht der Zusammensetzung oder Zubereitung.

Aus dem vorstehenden Text ergibt sich, dass ein weiterer Aspekt der vorliegenden Erfindung auch ein Verfahren zum Erzeugen, Modifizieren oder Verstärken eines Geschmacks, insbesondere eines Umami-Geschmacks, in einer der Ernährung oder dem Genuss dienenden (i) gebrauchs- oder verzehrfertigen Zubereitung oder (ii) Halbfertigware betrifft. Ein solches erfindungsgemäßes Verfahren umfasst den folgenden Schritt:
Vermischen einer geschmacklich wirksamen Menge einer oder mehrerer Verbindungen der Formel (I) oder (II), oder einer erfindungsgemäßen Zusammensetzung mit einem oder mehreren weiteren Bestandteilen der (i) verzehrfertigen Zubereitung oder der (ii) Halbfertigware
   und/oder
   Applizieren einer geschmacklich wirksamen Menge einer oder mehrerer Verbindungen der Formel (I) oder (II) oder einer erfindungsgemäßen Zusammensetzung auf einen oder mehrere weitere Bestandteile der (i) verzehrfertigen Zubereitung oder der (ii) Halbfertigware
   und/oder
   Einbetten einer geschmacklich wirksamen Menge einer oder mehrerer Verbindungen der Formel (I) oder (II) oder einer erfindungsgemäßen Zusammensetzung in ein Hüll- oder Matrixmaterial.

Entsprechend dem oben gesagten betrifft ein weiterer Aspekt der Erfindung die neuen, unter die allgemeine Formel (I) und (II) fallenden Verbindungen. Dementsprechend ist Bestandteil der Erfindung eine Verbindung der Formel (I) oder Mischung von Verbindungen der Formel (I) 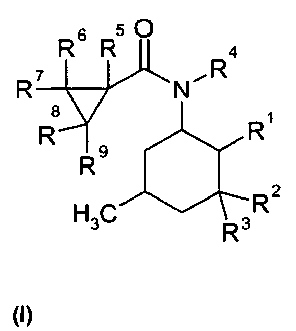 wobei gilt:
- R¹, R², R³: bedeuten jeweils unabhängig voneinander Wasserstoff oder einen Alkylrest von 1 bis 3 C-Atomen, insbesondere Methyl, Ethyl, n-Propyl oder i-Propyl-, wobei mindestens ein Rest ungleich H ist;
- R⁴: bedeutet Wasserstoff, einen Alkylrest von 1 bis 6 C-Atomen, insbesondere Methyl, Ethyl, n-Propyl, iso-Propyl, n-Butyl, iso-Butyl, sec.-Butyl, tert.-Butyl, n-Pentyl, 1-Methyl-butyl, 2-Methylbutyl, 3-Methyl-butyl, 1,2-Dimethyl-propyl, 1,1-Dimethyl-propyl, 2,2-Dimethyl-propyl, n-Hexyl, 1-Methyl-pentyl, 2-Methyl-pentyl, 3-Methyl-pentyl, 4-Methyl-pentyl, 1-Ethyl-butyl, 2-Ethyl-butyl, 1,1-Dimethyl-butyl, 1,2-Dimethyl-butyl, 1,3-Dimethyl-butyl, 2,2-Dimethyl-butyl, 2,3-Dimethyl-butyl, 3,3-Dimethyl-butyl, 1-Ethyl-1-methyl-propyl, 1-Ethyl-2-methyl-propyl, 1,1,2-Trimethyl-propyl oder 1,2,2-Trimethyl-propyl, oder einen Alkenylrest von 2 bis 6 C-Atomen, insbesondere 1-Propenyl, 2-Propenyl, 1-Methyl-1-propenyl, 2-Methyl-1-propenyl oder 2-Methyl-2-propenyl;
- R⁵, R⁶, R⁷ R⁸: bedeuten jeweils unabhängig voneinander Wasserstoff oder Methyl;
- R⁹: bedeutet Wasserstoff, einen Alkylrest mit 4 bis 12 C-Atomen insbesondere n-Butyl, iso-Butyl, sec.-Butyl, tert.-Butyl, n-Pentyl, 1-Methyl-butyl, 2-Methyl-butyl, 3-Methyl-butyl, 1,2-Dimethylpropyl, 1,1-Dimethyl-propyl, 2,2-Dimethyl-propyl, n-Hexyl, 1-Methyl-pentyl, 2-Methyl-pentyl, 3-Methyl-pentyl, 4-Methylpentyl, 1-Ethyl-butyl, 2-Ethyl-butyl, 1,1-Dimethyl-butyl, 1,2-Dimethyl-butyl, 1,3-Dimethyl-butyl, 2,2-Dimethyl-butyl, 2,3-Dimethyl-butyl, 3,3-Dimethyl-butyl, 1-Ethyl-1-methyl-propyl, 1-Ethyl-2-methyl-propyl, 1,1,2-Trimethyl-propyl, 1,2,2-Trimethylpropyl, n-Heptyl, n-Octyl, n-Nonyl, n-Decyl, n-Undecyl oder n-Dodecyl oder einen Alkenylrest mit 4 bis 12 C-Atomen, insbesondere 1-Methyl-1-propenyl, 2-Methyl-1-propenyl oder 2-Methyl-2-propenyl,
mit der Maßgabe, dass die Verbindungen der Formel (I), die unter die Formel (III) 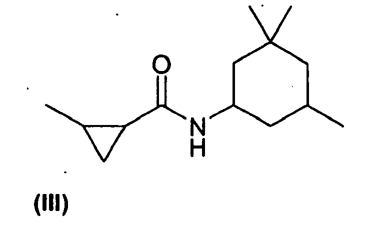 fallen, ausgenommen sind.

Verbindungen der Verbindung Formel (III) waren - in anderem Zusammenhang - bereits aus dem Stand der Technik bekannt (vergleiche weiter oben) und sind dementsprechend selbstverständlich ausgenommen.

Nach Auffassung der Anmelderin sind in der Offenlegungsschrift WO 2004/056745 keine Verbindungen ausreichend offenbart, die unter die allgemeine Formel I fallen. Sofern entgegen dieser Auffassung in der genannten Offenlegungsschrift doch konkrete Verbindungen als offenbart anzusehen sind, die unter die allgemeine Formel I wie oben definiert fallen, sind diese selbstverständlich nicht als Bestandteil der Erfindung aufzufassen. Zur Konkretisierung ist dementsprechend auf dem Wege der Verweisung die WO 2004/056745 in diese Anmeldung inkorporiert, dass gilt insbesondere auch für die Patentansprüche 1 und 2 in der genannten Offenlegungsschrift. Es ist vorsorglich bevorzugt, dass alle in der WO 2004/056745 als offenbart anzusehenden Verbindungen nicht Gegenstand der vorliegenden Erfindung sind.

Bevorzugt sind erfindungsgemäße Verbindungen der Formel (I), wobei die Verbindung oder die Verbindungen der Formel (I) eine Verbindung ist oder Verbindungen sind, ausgewählt aus der Gruppe bestehend aus:
(1 R,3S)-2,2-Dimethyl-3-(2-methyl-propenyl)-cyclopropancarbonsäure-((1 S,25,5R)-2-isopropyl-5-methyl-cydohexyl)-amid, (1 R,3S)-2,2-Dimethyl-3-(2-methyl-propenyl)-cyclopropancarbonsäure-((1 R,2R,5S)-2-isopropyl-5-methylcyclohexyl)-amid, (1S,3R)-2,2-Dimethyl-3-(2-methyl-propenyl)-cyclopropancarbonsäure-((1 S,2S,5R)-2-isopropyl-5-methyl-cyclohexyl)-amid, (1 S,3R)-2,2-Dimethyl-3-(2-methyl-propenyl)-cyclopropancarbonsäure-((1 R,2R,5S)-2-isopropyl-5-methyl-cyclohexyl)-amid, (1 S,3S)-2,2-Dimethyl-3-(2-methyl-propenyl)-cyclopropancarbonsäure-((1 S,2S,5R)-2-isopropyl-5-methylcyclohexyl)-amid, (1S,3S)-2,2-Dimethyl-3-(2-methyl-propenyl)-cyclopropancarbonsäure-((1 R,2R,5S)-2-isopropyl-5-methyl-cyclohexyl)-amid, (1 R,3R)-2,2-Dimethyl-3-(2-methyl-propenyl)-cyclopropancarbonsäure-((1S,25,5R)-2-isopropyl-5-methyl-cydohexyl)-amid, (1 R,3R)-2,2-Dimethyl-3-(2-methyl-propenyl)-cyclopropancarbonsäure-((1 R,2R,5S)-2-isopropyl-5-methylcyclohexyl)-amid.

Bevorzugte erfindungsgemäße Verbindungen oder erfindungsgemäße Mischungen von Verbindungen sind solche, die eine Verbindung oder Verbindungen der Formel (II) sind 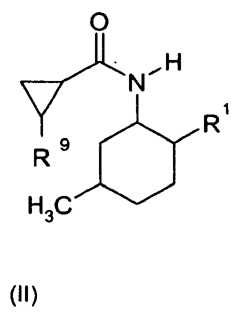 und wobei gilt:
- R¹: einen Alkylrest von 1 bis 3 C-Atomen, insbesondere Methyl-, Ethyl-, n-Propyl- oder i-Propyl-.
- R⁹: bedeutet Wasserstoff, einen Alkylrest mit 4 bis 12 C-Atomen insbesondere n-Butyl, iso-Butyl, sec.-Butyl, tert.-Butyl, n-Pentyl, 1-Methyl-butyl, 2-Methyl-butyl, 3-Methyl-butyl, 1,2-Dimethylpropyl, 1,1-Dimethyl-propyl, 2,2-Dimethyl-propyl, n-Hexyl, 1-Methyl-pentyl, 2-Methyl-pentyl, 3-Methyl-pentyl, 4-Methylpentyl, 1-Ethyl-butyl, 2-Ethyl-butyl, 1,1-Dimethyl-butyl, 1,2-Dimethyl-butyl, 1,3-Dimethyl-butyl, 2,2-Dimethyl-butyl, 2,3-Dimethyl-butyl, 3,3-Dimethyl-butyl, 1-Ethyl-1-methyl-propyl, 1-Ethyl-2-methyl-propyl, 1,1,2-Trimethyl-propyl, 1,2,2-Trimethylpropyl, n-Heptyl, n-Octyl, n-Nonyl, n-Decyl, n-Undecyl oder n-Dodecyl oder einen Alkenylrest mit 4 bis 12 C-Atomen, insbesondere Methyl-1-propenyl, 2-Methyl-1-propenyl oder 2-Methyl-2-propenyl.

Erfindungsgemäß besonders bevorzugt sind Verbindungen oder Mischungen von Verbindungen der allgemeinen Formel (II) wie oben definiert, die ausgewählt sind aus der Gruppe bestehend aus:
Cyclopropancarbonsäure((1 R,2S,5R)-2-isopropyl-5-methyl-cyclohexyl)-amid,
Cyclopropancarbonsäure((1 S,2R,5S)-2-isopropyl-5-methyl-cyclohexyl)-amid,
Cyclopropancarbonsäure((1 S,2S,5R)-2-isopropyl-5-methyl-cyclohexyl)-amid,
Cyclopropancarbonsäure((1 R,2R,5S)-2-isopropyl-5-methyl-cyclohexyl)-amid,
Cyclopropancarbonsäure((1 R,2R,5R)-2-isopropyl-5-methyl-cyclohexyl)-amid,
Cyclopropancarbonsäure((1 S,2S,5S)-2-isopropyl-5-methyl-cyclohexyl)-amid,
Cyclopropancarbonsäure((1 S,2R,5R)-2-isopropyl-5-methyl-cyclohexyl)-amid und
Cyclopropancarbonsäure((1 R,2S,5S)-2-isopropyl-5-methyl-cyclohexyl)-amid.

Die Erfindung betrifft ferner ein Verfahren zur Herstellung einer Verbindung der Formeln (11) und/oder (12) bzw. Mischungen der Formeln (11) und/oder (12), umfassend die folgenden Schritte:
(a) Durchführen einer Leuckart-Wallach Reaktion (siehe beispielsweise Ann. Chem. 1893, 276, 296-313) ausgehend von enantiomerenreinen oder racemischen Menthon zu den entsprechenden Formamiden der Formel (III), 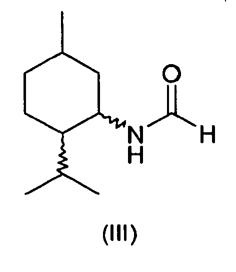
(b) Durchführen einer fraktionierten Kristallisation zu den entsprechenden Neo-Menthylformamiden, vorzugsweise in einer Diastereomerenreinheit ≥ 90 Gew.-%, bevorzugt ≥ 95 Gew.-% (racemisch oder in der jeweiligen D- bzw. L- Form Formeln (IV) und (V)), 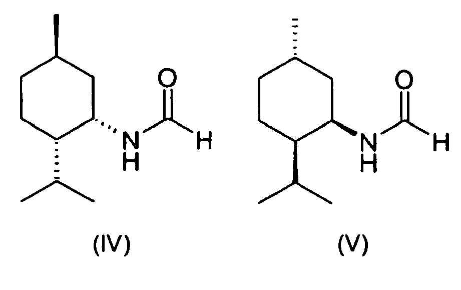
(c) Verseifung der Neo-Menthylformamide mit einer starken Säure, vorzugsweise einer Mineralsäure, insbesondere Salzäsure oder Schwefelsäure, mit einer Diastereomerenreinheit ≥ 90%, bevorzugt ≥ 95% (racemisch oder in der jeweiligen D- bzw. L- Form Formeln (VI) und (VII)) und 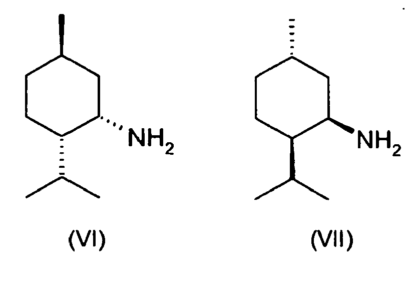
(d) Umsetzung des Amins entweder mit (i) Cyclopropancarbonsäurechlorid, vorzugsweise unter Schotten-Baumann Bedingungen oder mit (ii) Cyclopropancarbonsäure, vorzugsweise unter Borsäurekatalyse (vgl. Organic Process Research & Development 2007, 11, 1065-1068), wobei Diastereomerenreinheit vorzugsweise ≥ 90%, bevorzugt ≥ 95% (racemisch oder in der jeweiligen D- bzw. L- Form), beträgt zu (11) und/oder (12)

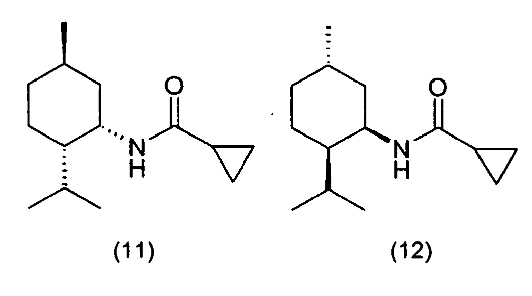

Die Erfindung wird nun nachfolgend anhand von Beispielen näher erläutert. Weitere Aspekte der vorliegenden Erfindung ergeben sich aus den beigefügten Patentansprüchen.

### Figurenbeschreibung:

**Fig. 1****:** Geschmacklicher Vergleich einer 1 : 1 Mischung aus Cyclopropancarbonsäure((1S,2S,5R)-2-isopropyl-5-methyl-cyclohexyl)amid (11) und Cyclopropancarbonsäure((1R,2R,5S)-2-isopropyl-5-methyl-cyclohexyl)amid (12) mit Natriumglutamat.

Der Geschmack eines 0,5% amerikanischen Rindfleischextrakts als Base (hellgraue Linie) wurde anhand einer Verkostung durch ein Panel ausgebildeter Testpersonen verglichen mit dem Geschmack erstens einer solchen Base, der 5 ppm einer Mischung bestehend aus Cyclopropancarbonsäure((1S,2S,5R)-2-isopropyl-5-methyl-cyclohexyl)amid (11) und Cyclopropancarbonsäure((1 R,2R,5S)-2-isopropyl-5-methyl-cyclohexyl)amid (12) zugesetzt wurde (durchgezogene Linie), und zweitens einer solchen Base, der 0,05 Gew.-% MSG zugesetzt wurde (gestrichelte Linie).

Die Testpersonen bewerteten die Stärke der angegebenen Geschmacksrichtungen durch Vergeben von Punktzahlen auf einer Skala von 0 (kein entsprechender Geschmack) bis 6 (sehr starker entsprechender Geschmack). Dargestellt sind die Mittelwerte der jeweiligen Punktzahlen.

### Beispiele:

Die nachfolgenden Beispiele erläutern die Erfindung ohne sie einzuschränken. Alle %-Angaben beziehen sich auf Gew.-% sofern nicht anders vermerkt.

Die als Ausgangsmaterial eingesetzten Menthylamine werden nach einer Vorschrift von Wallach et al. (Ann. Chem. 1893, 276, 296-313) aus den entsprechenden Menthonen in einer Reinheit ≥ 90%, bevorzugt ≥ 95%, dargestellt. Ein Gemisch aller möglichen isomeren Menthylamine kann nach oben genannter Vorschrift ohne entsprechende Kristallisation der Menthylformamide (ein Zwischenprodukt) in einer Reinheit von 99,3 Gew.-% erhalten werden (24,1% Menthylamin, 55,5% Neo-Menthylamin, 2,4% Iso-Menthylamin, 17,3% *Neo-Iso-*Menthylamin). Dabei können sowohl enantiomerenreine D- bzw. L-Menthone als auch eine racemische D/L-Menthonmischung verwandt werden. Alle eingesetzten Menthone können mit bis zu 25% der entsprechenden *Iso*-Menthone vermischt sein.

Die Darstellung einzelner enantiomeren- und diastereomerenreiner Menthylamine gelingt durch Umsetzung des entsprechenden Menthols zum Azid (Synthesis 1999, 8, 1373) und anschließender Reduktion mit LiAlH₄ (J. Am. Chem. Soc. 1962, 2925), siehe AAV1.

### Allgemeine Arbeitsvorschrift (AAV1): Amine ausgehend von Alkoholen

(a) Darstellung Chlormesylate: In einem 100 ml Rundkolben werden 30 - 35 mmol des entsprechenden Alkohls in 50 ml Pyridin gelöst und auf 0 °C abgekühlt. Anschließend werden langsam 1,1 - 1,5 mmol Chlormethansulfonylchlorid zugetropft und das Gemisch für für weitere 20 Minuten bei 0 °C gerührt. Dann wird mit Diethylether und Wasser verdünnt. Die etherische Phase wird abgetrennt und nachfolgend mit Wasser, 10%iger HCl Lösung, gesättigter Natriumcarbonatlösung und gesättigter Kochsalzlösung gewaschen. Nach Trocknen über Natriumsulfat und Entfernen des Lösungsmittels im Vakuum wird das Produkt entweder säulenchromatographisch (Diethylether/Pentan = 1:4) aufgereinigt oder direkt weiter umgesetzt.
(b) Darstellung der Azide: Das entsprechende Chlormesylat wird in 0,5 - 1,5 ml/mmol DMF aufgenommen und mit 2,0 - 3,0 Äquivalenten Natriumazid versetzt und 1 - 2 h auf 90°C erhitzt. Nach Abkühlen auf RT wird mit Diethylether und Wasser verdünnt und die organische Phase mit Wasser und gesättigter Kochsalzlösung gewaschen. Nach Trocknen über Natriumsulfat wird das Produkt am Rotationsverdampfer eingeengt und säulenchromatographisch (Diethylether/Pentan = 1:4) aufgereinigt.
(c) Reduktion zu Aminen: Das entsprechende Azid wird in 0,5-1,5 ml/mmol Diethylether aufgenommen und langsam zu einer Suspension aus 1,0 - 2,0 Äquivalenten Lithiumaluminium in 2,0 ml/mmol Diethylether getropft. Nach Refluxieren für 1 h unter Rückfluss wird die Reaktion nach Abkühlen mit 10%iger Natronlauge versetzt, bis ein weißer Niederschlag entsteht. Nach Zugabe von Natriumsulfat wird abfiltriert und das Lösungsmittel am Rotationsverdampfer entfernt. Der Rückstand wird in 10%iger Salzsäure aufgenommen und mit Diethylether gewaschen. Anschließend wird die wässrige Phase mit Kaliumcarbonat basisch gestellt und ebenfalls mit Diethylether extrahiert. Nach Trocknen über Natriumsulfat wird nach Entfernen des Lösungsmittels am Rotationsverdampfer das entsprechende Amin als enantiomeren-/ diastereomerenreine Verbindung erhalten.

### Allgemeine Arbeitsvorschrift (AAV2): Umsetzung mit Säurechloriden

Zu der Lösung eines entsprechenden Amins sowie 2,0 - 3,5 Äquivalenten Triethylamin in DMC werden unter Kühlung langsam 1,1 - 1,5 Äquivalente des entsprechenden Säurechlorides zugetropft. Die Mischung wird auf RT erwärmt und für 4 h gerührt. Anschließend wird mit Dichlormethan verdünnt und mit 10%iger Salzsäure und gesättigter Natriumhydrogencarbonatlösung gewaschen. Nach Trocknen über Natriumsulfat und anschließender Entfernung des Lösungsmittels erfolgt die Aufreinigung durch Umkristallisation oder Chromatographie.

### Synthesebeispiel 1: Cyclopropancarbonsäure ((1S,2S,5R)-2-isopropyl-5-methylcyclohexyl)-amid (11)

Ausgehend von enantiomerenreinem (L)-Menthol wurde durch Umsetzung analog **AAV 1** enantiomerenreines (L)-Neomenthylamin gewonnen, welches nach **AAV 2** mit Cyclopropancarbonsäurechlorid zum genannten Produkt umsetzt wurde. Die Aufreinigung des Produkts erfolgte durch Kristallisation (Pentan/Dichlormethan).
Analysendaten:
**¹H-NMR (400 MHz, CDCl₃):** 0.71 (m, 2H); 0.86 - 092 (m, 9H); 0.93 - 1.09 (m, 5H); 1.28 - 1.40 (m, 2H); 1.47 (m, 1 H); 1.75 (m, 1 H); 1.82 - 1.89 (m, 2H); 4.37 (m, 1 H); 5.63 (bd, J = 8.4 Hz, 1 H) ppm.
**¹³C-NMR (100 MHz, CDCl₃):** 6.9 (CH₂); 15.9 (CH); 20.9 (CH₃); 21.0 (CH₃); 22.3 (CH₃); 25.5 (CH₂); 26.9 (CH); 29.6 (CH); 34.8 (CH₂); 40.2 (CH2); 46.1 (CH); 46.3 (CH); 172.5 (C=O) ppm.

**Massenspektrum (EI):** m/z (%) = 223 (M^{·+}, 13); 180 (14); 138 (55); 112 (22); 99 (12); 98 (19) 95 (21); 86 (100); 81 (14); 70 (61); 69 (50); 55 (18); 43 (18); 41 (42).

### Synthesebeispiel 2: Cyclopropancarbonsäure((1 R,2R,5S)-2-isopropyl-5-methylcyclohexyl)-amid (12)

Ausgehend von enantiomerenreinem (D)-Menthol wurde durch Umsetzung analog **AAV 1** enantiomerenreines (D)-Neomenthylamin gewonnen, welches nach **AAV 2** mit Cyclopropancarbonsäurechlorid zum genannten Produkt umsetzt wurde. Die Aufreinigung des Produkts erfolgte durch Kristallisation (Pentan/Dichlormethan).
Analysendaten:
**¹H-NMR (400 MHz, CDCl₃):** 0.71 (m, 2H); 0.87 - 0.91 (m, 9H); 0.93 - 1.09 (m, 5H); 1.28 - 1.41 (m, 2H); 1.47 (m, 1 H); 1.75 (m, 1 H); 1.82 - 1.89 (m, 2H); 4.38 (m, 1H);5.61 (bd, *J* ≈ 8 Hz, 1 H) ppm.
**¹³C-NMR (100 MHz, CDCl₃):** 6.9 (CH₂); 15.1 (CH); 20.9 (CH₃); 21.0 (CH₃); 22.3 (CH₃); 25.5 (CH₂); 26.9 (CH); 29.6 (CH); 34.8 (CH₂); 40.2 (CH2); 46.1 (CH); 46.3 (CH); 172.5 (C=O) ppm.
**Massenspektrum (EI):** m/z (%) = 223 (M^{·+}, 13); 180 (16); 138 (59); 112 (28); 99 (14); 98 (23); 86 (100); 81 (17); 70 (85); 69 (71); 55 (20); 43 (28); 41 (67); 39 (14).

### Synthesebeispiel 3: Cyclopropancaronsäure cyclohexylamid (nicht erfindungsgemäß)

Ausgehend Cyclohexylamin wurde durch Umsetzung mit Cyclopropancarbonsäurechlorid analog **AAV 2** das genannten Produkt erhalten. Die Aufreinigung des Produkts erfolgte durch Kristallisation (Hexan/Aceton).

Analysendaten:
**¹H-NMR (400 MHz, CDCl₃):** 0.70 (m, 2H); 0.94 (m, 2H); 1.08 - 1.22 (m, 3H); 1.28 - 1.41 (m, 3H); 1.61 (m, 1 H); 1.71 (m, 2H); 1.92 (m, 2H); 3.77 (m, 1 H); 5.74 (bs, 1 H) ppm.
**¹³C-NMR (100 MHz, CDCl₃):** 6.9 (CH₂); 14.8 (CH); 25.0 (CH₂); 25.6 (CH₂); 33.3 (CH₂); 48.3 (CH); 172.6 (C=O) ppm.
**Massenspektrum (EI):** m/z (%) = 167 (M^{·+}, 9); 124 (20); 99 (15); 98 (26); 86 (100); 72 (21); 70 (15); 69 (90); 67 (11); 56 (66); 55 (19); 44 (11); 43 (34); 41 (62); 39 (24).

### Synthesebeispiel 4A (Schotten-Baumann Variante): Cyclopropancarbonsäure ((1S,2S,5R)-2-isopropyl-5-methyl-cyclohexyl)-amid (11) / Cyclopropancarbonsäure((1 R,2R,5S)-2-isopropyl-5-methyl-cyclohexyl)-amid (12)

(D/L)-Neo-Menthylamin wurde in einer Mischung aus 1,1 Äquivalenten einer 3 Gew.-% Natriumhydroxidlösung und 0,5 - 1,0 ml/mmol Aceton bei Raumtemperatur vorgelegt und mit einer Spritzenpumpe mit 1,0 Äquivalenten Cyclopropancarbonsäurechlorid versetzt. Nach beendeter Zugabe (das Produkt ist bereits teilweise ausgefallen) wurde auf 50°C erwärmt, wobei der Feststoff erneut in Lösung geht. Nun wurde mit 2,5 - 3,0 ml/mmol Leitungswasser versetzt und auf 5°C abgekühlt. Der Feststoff wurde abfiltriert und gründlich mit Phosphatpuffer und Leitungswasser gewaschen, bevor das Produkt bis zur Massenkonstanz im Trockenschrank bei 65°C getrocknet wurde.
Die NMR und MS-Daten entsprachen denen der Synthesebeispiele 1 und 2.

### Synthesebeispiel 4B (Borsäurekatalyse): Cyclopropancarbonsäure ((1S,2S,5R)-2-isopropyl-5-methyl-cyclohexyl)-amid (11) / Cyclopropancarbonsäure((1 R,2R,5S)-2-isopropyl-5-methyl-cyclohexyl)-amid (12)

Cyclopropancarbonsäure wurde in 1,5 - 2,5 ml/mmol Toluol bei Raumtemperatur gelöst und mit 0,1 Äquivalenten Borsäure sowie 1,05 Äquivalenten (D/L)-Neo-Menthylamin versetzt. Die Reaktionsmischung wurde nun am Wasserabscheider zum Rückfluss erhitzt. Wurde kein Wasser mehr übergetrieben und war die Reaktion beendet (ca. 16 h) wurde die Reaktionsmischung mit weiteren 1,5 - 2,5 ml/mmol Toluol verdünnt und auf Raumtemperatur abgekühlt. Anschließend wurde mit 10%iger Salzsäure, Leitungswasser und gesättigter Natriumhydrogencarbonatlösung gewaschen. Nach Trocknen über Natriumsulfat wurde das Toluol abdestilliert und der Rückstand in Pentan suspendiert. Der Feststoff wurde abfiltriert und mehrfach mit Pentan gewaschen und anschließend bei 40 °C im Vakuum getrocknet.
Die NMR und MS-Daten entsprachen denen der Synthesebeispiele 1 und 2.

In den folgenden Beispielen wurde eine Mischung aus Cyclopropancarbonsäure ((1 S,2S,5R)-2-isopropyl-5-methyl-cyclohexyl)-amid (11) und Cyclopropancarbonsäure((1 R,2R,5S)-2-isopropyl-5-methyl-cyclohexyl)-amid (12) eingesetzt, wie sie gemäß Beispiel 4A erhalten werden kann. Da es sich dabei um ein Racemat handelte, ist das Gewichtsverhältnis von (11) : (12) gleich 1 : 1.

### Beispiel 1: Sprühgetrocknete Zusammensetzung zur Erzeugung eines Umami-Geschmacks

**1.1**

| **Bestandteil** | **Anteil** |
|---|---|
| Mischung aus 11,2 % Cyclopropancarbonsäure((1R,2S,5R)-2-isopropyl-5-methyl-cyclohexyl)-amid (9), 11,2 % Cyclopropancarbonsäure((1S,2R,5S)-2-isopropyl-5-methyl-cyclohexyl)-amid (10), 31,4 % Cyclopropancarbonsäure((1S,2S,5R)-2-isopropyl-5-methyl-cyclohexyl)-amid (11), 31,4 % Cyclopropancarbonsäure((1R,2R,5S)-2-isopropyl-5-methyl-cyclohexyl)-amid (12), 0,6 % Cyclopropancarbonsäure((1R,2R,5R)-2-isopropyl-5-methyl-cyclohexyl)-amid (13), 0,6 % Cyclopropancarbonsäure((1S,2S,5S)-2-isopropyl-5-methyl-cyclohexyl)-amid (14), 6,8 % Cyclopropancarbonsäure((1S,2R,5R)-2-isopropyl-5-methyl-cyclohexyl)-amid (15) und 6,8 % Cyclopropancarbonsäure((1R,2S,5S)-2-isopropyl-5-methyl-cyclohexyl)-amid (16) | 2 g |
| Maltodextrin | 98 g |

**1.2**

| **Bestandteil** | **Anteil** |
|---|---|
| Mischung aus Cyclopropancarbonsäure ((1S,2S,5R)-2-isopropyl-5-methyl-cyclohexyl)-amid (11) / Cyclopropancarbonsäure((1R,2R,5S)-2-isopropyl-5-methyl-cyclohexyl)-amid (12) (1 : 1) | 4 g |
| Maltodextrin | 96 g |

Die Bestandteile wurden in einer Mischung aus Ethanol und demineralisiertem Wasser gelöst und anschließend sprühgetrocknet.

### Beispiel 2: Aromakomposition

| **Inhaltsstoff** | **Anteil** |
|---|---|
| 10 Gew.-% Pellitorin in 1,2-Propylenglycol/Diethylmalonat (1:1) | 0,25 g |
| Mischung aus Cyclopropancarbonsäure ((1S,2S,5R)-2-isopropyl-5-methyl-cyclohexyl)-amid (11) / Cyclopropancarbonsäure((1R,2R,5S)-2-isopropyl-5-methyl-cyclohexyl)-amid (12) | 10,00 g |
| Hesperetin | 2,00 g |
| Phloretin | 1,50 g |
| Propylenglycol | 86,25 g |

Die Inhaltsstoffe (Stoffe bzw. Lösungen) wurden in den oben angegebenen Mengenverhältnissen gemischt und dann mit Propylenglycol aufgenommen und durch leichtes Erwärmen vollständig gelöst.

### Beispiel 3: Würzmittel, enthaltend Verbindung zur Erzeugung eines Umami-Geschmacks und eine Aromakomposition

| **Teil** | **Bestandteil** | **Anteil** |
|---|---|---|
| A | (1 : 1)-Mischung aus (11) + (12) | 0,02 g |
| | Natriumchlorid | 15,0 g |
| B | Senfsamenmehl | 5,0 g |
| | Senfaroma | 0,1 g |

Teil A wurde eingewogen. Es wurden 290 ml Wasser vorgelegt und unter Rühren Teil A zugegeben und gelöst. Die Lösung wird mit Wasser auf 1,84 kg verdünnt (pH 6,0) und anschließend gefriergetrocknet (Eutektischer Punkt: -15°C; Arbeitsvakuum: 0,52 mbar; Stellflächentemperatur: -5°C bis +25°C). Das Produkt wird mit Senfsamenmehl und dem Senfaroma aus Teil B gemischt und zu einem Würzmittel konfektioniert.

### Beispiel 4: Instantsuppe, Typ Lauch-Creme

| **Bestandteil** | **Vergleichszubereitung A** | **Erfindungsgemäße Zubereitung B** | **Erfindungsgemäße Zubereitung C** | **Erfindungsgemäße Zubereitung D** |
|---|---|---|---|---|
| Kartoffelstärke | 20,0 g | 21,0 g | 21,0 g | 21,0 g |
| Fettpulver | 25,0 g | 26,0 g | 26,0 g | 26,0 g |
| Lactose | 20,0 g | 21,0 g | 21,0 g | 21,0 g |
| Maltodextrin | 11,73 g | 11,727 g | 11,70 g | 11,43 g |
| Kochsalz | 8,0 g | 8,0 g | 8,0 g | 8,0 g |
| Natriumglutamat | 3,0 g | - | - | - |
| Spinatpulver | 2,0 g | 2,0 g | 2,0 g | 2,0 g |
| Grünes Lauchpulver | 2,0 g | 2,0 g | 2,0 g | 2,0 g |
| Citronensäure, als Pulver | 0,3 g | 0,3 g | 0,3 g | 0,3 g |
| Gehärtetes Pflanzenfett | 3,0 g | 3,0 g | 3,0 g | 3,0 g |
| Gefriergetrockneter Lauch | 1,0 g | 1,0 g | 1,0 g | 1,0 g |
| Huhnaroma | 1,0 g | 1,0 g | 1,0 g | 1,0 g |
| Würzmischung Typ "grüner Lauch", Pulver | 2,0 g | 2,0 g | 2,0 g | 2,0 g |
| Würzmischung, Typ "gekochte Zwiebel" | 0,6 g | 0,6 g | 0,6 g | 0,6 g |
| Hefe-Würzmischung, Typ "Gemüsebrühe", Pulver | 0,3 g | 0,3 g | 0,3 g | 0,3 g |
| Curcuma-Extrakt | 0,07 g | 0,07 g | 0,07 g | 0,07 g |
| (1 : 1)-Mischung aus (11)+(12) | - | 0,006 g | 0,06 g | 0,60 g |

5 g der jeweiligen Pulvermischung wurden mit je 100 ml heißem Wasser aufgegossen, um eine verzehrfertige Suppe zu erhalten.

Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Beispiel 5: Instantsuppe, Typ Hühnersuppe mit Nudeln

| **Bestandteil** | **Vergleichszubereitung A** | **Erfindungsgemäße Zubereitung B** | **Erfindungsgemäße Zubereitung C** | **Erfindungsgemäße Zubereitung D** |
|---|---|---|---|---|
| Stärke | 16 g | 17,2 g | 17,2 g | 17,2 g |
| Kochsalz | 7 g | 7 g | 7 g | 7 g |
| Saccharose, raffiniert | 3,2 g | 3,2 g | 3,2 g | 3,2 g |
| Natriumglutamat | 3,2 g | - | - | - |
| Natriuminosinat / Natriumguanylat im Verhältnis 1:1 | 0,8 g | 0,8 g | 0,8 g | 0,8 g |
| Säurehydrolysiertes Pflanzenprotein | 8,0 g | 8,0 g | 8,0 g | 8,0 g |
| Fettpulver | 2,0 g | 2,0 g | 2,0 g | 2,0 g |
| Gemüsefett, sprühgetrocknet | 1,0 g | 1,0 g | 1,0 g | 1,0 g |
| Gefriergetrocknetes Hühnerfleisch, in Stückchen | 2,0 g | 2,0 g | 2,0 g | 2,0 g |
| Suppen-Nudeln | 32,0 g | 33,0 g | 33,0 g | 33,0 g |
| Maltodextrin | 12,16 g | 13,157 g | 13,13 g | 12,86 g |
| Chinesisches Gemüse, gefriergetrocknet | 4,6 g | 4,6 g | 4,6 g | 4,6 g |
| Huhnaroma | 8,0 g | 8,0 g | 8,0 g | 8,0 g |
| Lebensmittelfarbstoff Riboflavin | 0.04 g | 0.04 g | 0.04 g | 0.04 g |
| (1 : 1)-Mischung aus (11) + (12) | - | 0,006 g | 0,06 g | - |
| Aromenkomposition nach A | - | - | - | 0,60 g |

4,6 g der jeweiligen Pulvermischung wurden 10 Minuten in je 100 ml Wasser gekocht, um eine verzehrfertige Suppe zu erhalten.

Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Beispiel 6: Würzmischung, Typ "Pfeffer":

| **Bestandteil** | **Vergleichszubereitung A** | **Erfindungsgemäße Zubereitung B** | **Erfindungsgemäße Zubereitung C** | **Erfindungsgemäße Zubereitung D** |
|---|---|---|---|---|
| Milchprotein | 0,8 g | 0,8 g | 0,8 g | 0,8 g |
| Johannisbrotkernmehl | 2,0 g | 2,0 g | 2,0 g | 2,0 g |
| Maisstärke | 25,0 g | 26,994 g | 26,94 g | 26,4 g |
| Kochsalz | 14,0 g | 15,0 g | 15,0 g | 15,0 g |
| Paprikapulver | 12,0 g | 13,0 g | 13,0 g | 13,0 g |
| Tomatenpulver | 12,0 g | 13,0 g | 13,0 g | 13,0 g |
| Saccharose | 4,0 g | 4,0 g | 4,0 g | 4,0 g |
| Knoblauchpulver | 0,5 g | 0,5 g | 0,5 g | 0,5 g |
| Gehärtetes Pflanzenfett | 8,0 g | 8,0 g | 8,0 g | 8,0 g |
| Fettpulver | 10,0 g | 11,0 g | 11,0 g | 11,0 g |
| Natriumglutamat | 6,0 g | - | - | - |
| Lebensmittelfarbstoff Rote Bete und Paprika | 2,0 g | 2,0 g | 2,0 g | 2,0 g |
| Aroma Typ "Pfeffer" | 2,0 g | 2,0 g | 2,0 g | 2,0 g |
| Aroma Typ "Pizza" | 1,2 g | 1,2 g | 1,2 g | 1,2 g |
| Aroma Typ "Tomate" | 0,4 g | 0,4 g | 0,4 g | 0,4 g |
| Extrakt aus schwarzem Pfeffer | 0,1 g | 0,1 g | 0,1 g | 0,1 g |
| (1 : 1)-Mischung aus (11)+(12) | - | 0,012 g | 0,12 g | 1,20 g |

Jeweils 100 g Nackensteak vom Schwein wurde mit jeweils 1,7 g der Zubereitungen A, B, C und D gleichmäßig bestreut und gebraten. Bei der Verkostung durch ein Panel ausgebildeter Testpersonen wurden die Vergleichszubereitung A und die erfindungsgemäße Zubereitung C gleich bewertet. Bei der erfindungsgemäßen Zubereitung B wurden Umami-Geschmack (und Mundfülle) als wahrnehmbar, jedoch schwächer gegenüber den Zubereitungen A und C beschrieben. Die erfindungsgemäße Zubereitung D wurde hinsichtlich Umami-Geschmack (und Mundfülle) als sehr ausgeprägt und deutlich stärker als die Zubereitungen A und C bewertet.

### Beispiel 7: Tomatenketchup

| **Bestandteil** | **Vergleichszubereitung A** | **Erfindungsgemäße Zubereitung B** | **Erfindungsgemäße Zubereitung C** | **Erfindungsgemäße Zubereitung D** |
|---|---|---|---|---|
| Natriumglutamat | 6 g | - | - | - |
| Kochsalz | 2 g | 2 g | 2 g | 2 g |
| Stärke, Farinex WM 55 | 1 g | 1 g | 1 g | 1 g |
| Sucrose | 12 g | 12 g | 12 g | 8,4 g |
| Tomaten-Konzentrat 2-fach | 36 g | 36 g | 36 g | 32 g |
| Glucosesirup 80 Brix | 18 g | 18 g | 18 g | 18 g |
| Branntweinessig 10% | 7 g | 7 g | 7 g | 3 g |
| Wasser | 18 g | 23.8 g | 23.5 g | 34,7 g |
| Phloretin 2,5% in 1,2-Propylenglycol | - | - | - | 0,2 g |
| Hesperetin 2,5%ig in 1,2-Propylenglycol | - | - | - | 0,2 g |
| (1 : 1)-Mischung aus (11)+(12) | - | 0.2g | 0.5g | 0.5g |

Die Inhaltsstoffe wurden in der angegebenen Reihenfolge gemischt und das fertige Ketchup mit Hilfe eines Rührwerkes homogenisiert, in Flaschen abgefüllt und sterilisiert.

### Beispiel 8: Bouillon

| **Bestandteil** | **Vergleichszubereitung A** | **Natriumglutamatreduzierte Vergleichszubereitung B** | **Erfindungsgemäße Natriumglutamatreduzierte Zubereitung C** | **Erfindungsgemäße Natriumglutamatfreie Zubereitung D** |
|---|---|---|---|---|
| Fettpulver | 8,77 g | 8,77 g | 8,77 g | 8,77 g |
| Natriumglutamat | 8,77 g | 5 g | 5 g | - |
| Hefeextrakt Pulver | 12,28 g | 12,28 g | 12,28 g | 12,28 g |
| Kochsalz | 29,83 g | 29,83 g | 29,83 g | 29,83 g |
| Maltodextrin | 37,28 g | 37,28 g | 37,28 g | 37,28 g |
| Natürlicher Gemüseextrakt | 3,07 g | 3,07 g | 3,07 g | 3,07 g |
| (1 : 1)-Mischung aus (11) + (12) | - | - | 0,10 g | 0,24 g |

15 g der jeweiligen Pulvermischung wurden mit je 1000 ml heißem Wasser aufgegossen. Bei der Verkostung durch ein Panel ausgebildeter Testpersonen wurden die Vergleichszubereitung A und die erfindungsgemäße Natriumglutamat-freie Zubereitung D gleich bewertet. Bei der erfindungsgemäßen Zubereitung C wurde ebenfalls eine deutliche Verbesserung bezüglich Umami-Geschmack (und Mundfülle) gegenüber der Natriumglutamat-reduzierten Vergleichszubereitung B wahrgenommen.

### Beispiel 9: Reaktionsaroma

| **Inhaltsstoff** | **Einsatz [in Gramm]** |
|---|---|
| L-Alanin | 41,0 |
| L-Asparaginsäure | 123,0 |
| Bernsteinsäure | 4,7 |
| Calciumchlorid Dihydrat | 7,0 |
| L-Cystein·HCl Monohydrat | 11,0 |
| Dikaliumphosphat | 6,0 |
| Fructose gemahlen | 1,0 |
| L-lsoleucin | 1,6 |
| Kaliumchlorid | 228,0 |
| L-Leucin | 1,6 |
| L-Lysin·HCl | 3,6 |
| Magnesiumchlorid Hexahydrat | 19,0 |
| Maltodextrin | 49,0 |
| L-Phenylalanin | 2,0 |
| L-Prolin | 74,0 |
| L-Serin | 6,5 |
| L-Threonin | 3,0 |
| L-Valin | 9,0 |
| Wasser | 399,0 |
| (1 : 1)-Mischung aus (11) + (12), 20 Gew.-% in EtOH | 10,0 |

Alle Komponenten wurden bei 40°C gemischt und anschließend bei 85°C für 10 Minuten erhitzt (Rückflussreaktion). Nach dem Abkühlen auf 40°C wurde mit Kalilauge auf pH 5 eingestellt. Dieses Umami-Reaktionsaroma wurde analog in Beispiel 5 anstelle der (1 : 1)-Mischung aus (11)+(12) in die Bouillon - Zubereitungen C bzw. D des Anwendungsbeispiels 5 eingearbeitet, wobei in Zubereitung C 12 g und in Zubereitung D 28 g des Umami-Reaktionsaromas verwendet wurden.

### Beispiel 10: Würzmischung für Kartoffelchips

| **Bestandteil** | **Vergleichszubereitung A** | **Natriumglutamatreduzierte Vergleichszubereitung B** | **Erfindungsgemäße Natriumglutamatreduzierte Zubereitung C** | **Erfindungsgemäße Natriumglutamatfreie Zubereitung D** |
|---|---|---|---|---|
| Natriumglutamat | 3,50 g | 2 g | 2 g | - |
| Käsepulver | 10,00 g | 10,00 g | 10,00 g | 10,00 g |
| Knoblauchpulver | 2,00 g | 2,00 g | 2,00 g | 2,00 g |
| Molkenpulver | 38,86 g | 38,86 g | 38,86 g | 38,86 g |
| Würzextraktöl | 0,20 g | 0,20 g | 0,20 g | 0,20 g |
| Paprikapulver | 9,80 g | 9,80 g | 9,80 g | 9,80 g |
| Kochsalz | 21,00 g | 21,00 g | 21,00 g | 21,00 g |
| Tomatenpulver | 9,00 g | 9,00 g | 9,00 g | 9,00 g |
| Trockenaroma | 2,50 g | 2,50 g | 2,50 g | 2,50 g |
| Siliciumdioxid | 0,02 g | 0,02 g | 0,02 g | 0,02 g |
| Pflanzenöl | 0,02 g | 0,02 g | 0,02 g | 0,02 g |
| Zwiebelpulver | 3,00 g | 3,00 g | 3,00 g | 3,00 g |
| Sahne Aromakonzentrat | 0,03 g | 0,03 g | 0,03 g | 0,03 g |
| Käse Aroma | 0,03 g | 0,03 g | 0,03 g | 0,03 g |
| Tomaten Aromakonzentrat | 0,04 g | 0,04 g | 0,04 g | 0,04 g |
| Sprühgetrocknete Zusammensetzung gemäß Beispiel 1.1 | - | - | 2,80 g | 5,40 g |

6 g der Würzmischung wurden auf 94 g Kartoffelchips aufgezogen. Bei der Verkostung durch ein Panel ausgebildeter Testpersonen wurden die Vergleichszubereitung A und die erfindungsgemäße Natriumglutamat-freie Zubereitung D gleich bewertet. Bei der erfindungsgemäßen Zubereitung C wurde ebenfalls eine deutliche Verbesserung bezüglich Umami-Geschmack (und Mundfülle) gegenüber der Natriumglutamat-reduzierten Vergleichszubereitung B wahrgenommen.

### Beispiel 11: Weiße Soße

| **Bestandteil** | **Vergleichszubereitung A** | **Natriumglutamat-reduzierte Vergleichszubereitung B** | **Erfindungsgemäße Natriumglutamat-reduzierte Zubereitung C** | **Erfindungsgemäße Natriumglutamat-freie Zubereitung D** |
|---|---|---|---|---|
| Maltodextrin | 26,28 g | 26,28 g | 26,28 g | 26,28 g |
| Kochsalz | 7,50 g | 7,50 g | 7,50 g | 7,50 g |
| Natriumglutamat | 2,00 g | 0,80 g | 0,80 g | - |
| Pflanzenfett | 5,00 g | 5,00 g | 5,00 g | 5,00 g |
| Pfeffer, weiß | 0,02 g | 0,02 g | 0,02 g | 0,02 g |
| Zwiebelpulver | 1,50 g | 1,50 g | 1,50 g | 1,50 g |
| vorverkleisterte Maisstärke | 30,00 g | 30,00 g | 30,00 g | 30,00 g |
| Fettpulver | 27,70 g | 27,70 g | 27,70 g | 27,70 g |
| Sprühgetrocknete Zusammensetzung gemäß Beispiel 1.2 | - | - | 2,00 g | 3,60 g |

90 g der Soßenmischung wurden mit 1000 ml heißem Wasser aufgegossen und mit dem Schneebesen kräftig verrührt. Bei der Verkostung durch ein Panel ausgebildeter Testpersonen wurden die Vergleichszubereitung A und die erfindungsgemäße Natriumglutamat-freie Zubereitung D gleich bewertet. Bei der erfindungsgemäßen Zubereitung C wurde ebenfalls eine deutliche Verbesserung bezüglich Umami-Geschmack (und Mundfülle) gegenüber der Natriumglutamat-reduzierten Vergleichszubereitung B wahrgenommen.

### Beispiel 12: Braune Soße

| **Bestandteil** | **Vergleichszubereitung A** | **Natriumglutamat-reduzierte Vergleichszubereitung B** | **Erfindungsgemäße Natriumglutamat-reduzierte Zubereitung C** | **Erfindungsgemäße Natriumglutamat-freie Zubereitung D** |
|---|---|---|---|---|
| Stärke | 40,00 g | 40,00 g | 40,00 g | 40,00 g |
| Maltodextrin | 33,10 g | 33,10 g | 33,10 g | 33,10 g |
| Kochsalz | 6,00 g | 6,00 g | 6,00 g | 6,00 g |
| Zuckerkulör, sprühgetrocknet | 5,00 g | 5,00 g | 5,00 g | 5,00 g |
| Hefeextraktpulver | 3,00 g | 3,00 g | 3,00 g | 3,00 g |
| Natriumglutamat | 2,00 g | 1,30 g | 1,30 g | - |
| Zucker | 0,50 g | 0,50 g | 0,50 g | 0,50 g |
| Fettpulver | 5,00 g | 5,00 g | 5,00 g | 5,00 g |
| Tomatenpulver | 3,00 g | 3,00 g | 3,00 g | 3,00 g |
| Natürlicher Gemüseextrakt | 1,00 g | 1,00 g | 1,00 g | 1,00 g |
| Zwiebelextrakt | 0,30 g | 0,30 g | 0,30 g | 0,30 g |
| Pfefferextrakt | 0,10 g | 0,10 g | 0,10 g | 0,10 g |
| Trockenaroma | 1,00 g | 1,00 g | 1,00 g | 1,00 g |
| Sprühgetrocknete Zusammensetzung gemäß Beispiel 1.1 | - | - | 1,40 g | 4,00 g |

90 g der Soßenmischung wurden mit 1000 ml heißem Wasser aufgegossen und mit dem Schneebesen kräftig verrührt. Bei der Verkostung durch ein Panel ausgebildeter Testpersonen wurden die Vergleichszubereitung A und die erfindungsgemäße Natriumglutamat-freie Zubereitung D gleich bewertet. Bei der erfindungsgemäßen Zubereitung C wurde ebenfalls eine deutliche Verbesserung bezüglich Umami-Geschmack (und Mundfülle) gegenüber der Natriumglutamat-reduzierten Vergleichszubereitung B wahrgenommen.

### Beispiel 13: Tomatensuppe

| **Bestandteil** | **Vergleichszubereitung A** | **Natriumglutamat-reduzierte Vergleichszubereitung B** | **Erfindunggemäße Natriumglutamatreduzierte Zubereitung C** | **Erfindungsgemäße Natriumglutamatfreie Zubereitung D** |
|---|---|---|---|---|
| Wasser | 50,65 g | 50,65 g | 50,65 g | 50,65 g |
| Pflanzenöl | 5,50 g | 5,50 g | 5,50 g | 5,50 g |
| Tomatenpaste | 24,00 g | 24,00 g | 24,00 g | 24,00 g |
| Sahne | 1,05 g | 1,05 g | 1,05 g | 1,05 g |
| Zucker | 2,00 g | 2,00 g | 2,00 g | 2,00 g |
| Kochsalz | 1,70 g | 1,70 g | 1,70 g | 1,70 g |
| Natriumglutamat | 0,40 g | 0,25 g | 0,25 g | - |
| Weizenmehl | 5,50 g | 5,50 g | 5,50 g | 5,50 g |
| Stärke | 1,20 g | 1,20 g | 1,20 g | 1,20 g |
| gewürfelte Toma-ten | 8,00 g | 8,00 g | 8,00 g | 8,00 g |
| Sprühgetrocknete Zusammensetzung gemäß Beispiel 1.2 | - | - | 0,40 g | 0,80 g |

Die festen Bestandteile wurden eingewogen, gemischt und dem Wasser hinzugefügt. Das Pflanzenöl wurde zudosiert und die Tomatenpaste hinzugegeben. Bei der Verkostung durch ein Panel ausgebildeter Testpersonen wurden die Vergleichszubereitung A und die erfindungsgemäße Natriumglutamat-freie Zubereitung D gleich bewertet. Bei der erfindungsgemäßen Zubereitung C wurde ebenfalls eine deutliche Verbesserung bezüglich Umami-Geschmack (und Mundfülle) gegenüber der Natriumglutamat-reduzierten Vergleichszubereitung B wahrgenommen.

### Beispiel 14: Anwendung in einem zuckerfreien Kaugummi

| **Teil** | **Inhaltsstoff** | **Einsatz in Gew.-%** |
|---|---|---|
| A | Kaugummibase, Company "Jagum T" | 30,00 |
| B | Sorbit, pulverisiert | 39,00 |
| | Isomalt® (Palatinit GmbH) | 9,50 |
| | Xylit | 2,00 |
| | Mannit | 3,00 |
| | Aspartam® | 0,10 |
| | Acesulfam® K | 0,10 |
| | Emulgum® (Colloides Naturels, Inc.) | 0,30 |
| C | Sorbitol, 70 % | 14,00 |
| | Glycerin | 1,00 |
| D | Aromakomposition, enthaltend 0,1 Gew.-% 2E,4E-Decadiensäure-N-isobutylamid und 20 Gew.-% an Cyclopropancarbonsäure ((1S,2S,5R)-2-isopropyl-5-methyl-cyclohexyl)-amid (11) | 1 |

Teile A bis D werden gemischt und intensiv geknetet. Die Rohmasse kann z.B. in Form von dünnen Streifen zu verzehrfertigen Kaugummis verarbeitet werden.

### Beispiel 15: Anwendung in einem Grünteegetränk

| **Inhaltsstoff** | **Gewichtsteile** |
|---|---|
| Grünteekonzentrat | 18,00 |
| 1% ige Lösung einer equimolaren Mischung der Formeln (11) und (12) in Propylenglycol | 0,40 |
| entmineralisiertes Wasser | 81,40 |

Das Grünteekonzentrat wurde mit der 1 Gew.-%igen Lösung einer equimolaren Mischung der Formeln (1) und (2) in Propylenglycol vermischt. Anschließend wurde mit entmineralisiertem Wasser aufgefüllt und erneut gründlich durchmischt. Dann wurde das Produkt gefiltert, verbrauchsfertig verpackt und bei 118°C sterilisiert. Der Geschmack wurde durch ein Panel ausgebildeter Testpersonen als deutlich bevorzugt gegenüber der nicht aromatisierten Grünteebase bewertet.

### Beispiel 16: Rindfleischgewürz für Fertig-Nudeln

| **Inhaltsstoff** | **Gewichtsteile** |
|---|---|
| Rindsfettaroma | 5,00 |
| Zuckercouleur | 3,00 |
| Zitronensäure (wasserfrei) | 0,40 |
| Schnittlauch (entwässert) | 2,00 |
| Maltodextrin (ex Tapoica) | 10,30 |
| Mononatriumglutamat | 15,00 |
| Zwiebelpulver | 5,00 |
| Ribotide | 0,80 |
| Natriumchlorid | 45,65 |
| Zucker | 2,80 |
| Süßmolkepulver | 6,50 |
| Verbindungen der Formeln (11) und (12) | 0,05 |

Alle Inhaltsstoffe wurden vermischt bis sich eine homogene Mischung ergibt.

### Beispiel 17: Fertig-Nudeln mit Rindfleischgewürz

| **Teil** | **Inhaltsstoff** | **Gewichtsteile** |
|---|---|---|
| A | Weizenmehl | 62,00 |
| | Kartoffelstärke | 10,90 |
| B | Salz | 1,10 |
| | Guarkernmehl | 0,06 |
| | Natriumcarbonat | 0,07 |
| | Kaliumcarbonat | 0,25 |
| | Na₂H₂P₂O₇ | 0,07 |
| | Verbindungen der Formeln (11) und (12) | 0,05 |
| C | Wasser | 25,50 |

Eine Suspension der Zutaten B in Wasser wurde zu einer Mischung der Zutaten A gegeben und zu einem Teig geknetet. Nachdem der Teig für ca. 5 Minuten geruht hatte, wurde dieser mit Hilfe einer Nudelmaschine zu Platten verarbeitet, die in einem letzten Arbeitsschritt in eine übliche Form zurechtgeschnitten wurden. Die Nudeln waren nach einer Kochzeit von 3 Minuten verzehrfertig und wurden mit 8 g des Rindfleischgewürzes aus Anwendungsbeispiel 12 angerichtet.

## Patentansprüche

1. Verwendung einer Verbindung der Formel (I) oder einer Mischung von Verbindungen der Formel (I) 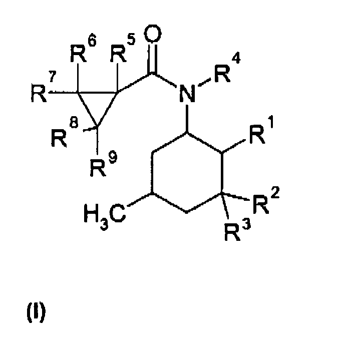 wobei gilt:
R¹, R², R³ bedeuten jeweils unabhängig voneinander Wasserstoff oder einen Alkylrest von 1 bis 3 C-Atomen, wobei mindestens ein Rest ungleich H ist;
R⁴ bedeutet Wasserstoff, einen Alkylrest von 1 bis 6 C-Atomen oder einen Alkenylrest von 2 bis 6 Atomen;
R⁵, R⁶, R⁷, R⁸ bedeuten jeweils unabhängig voneinander Wasserstoff oder Methyl;
R⁹ bedeutet Wasserstoff, einen Alkylrest mit 5 bis 12 C-Atomen oder einen Alkenylrest mit 5 bis 12 C-Atomen
als Geschmacksstoff oder Geschmacksstoffmischung.

2. Verwendung nach Anspruch 1, wobei die Verbindung oder die Verbindungen der Formel (I) eine Verbindung ist oder Verbindungen sind, ausgewählt aus der Gruppe bestehend aus:
(1R,3S)2,2-Dimethyl-3-(2-methyl-propenyl)-cyclopropancarbonsäure-((1S,2S,5R)-2-isopropyl-5-methyl-cyclohexyl)-amid (1), (1R3S)-2,2-Dimethyl-3-(2-methyl-propenyl)-cyclopropancarbonsäure-((1R,2R,5S)-2-isopropyl-5-methylcyclohexyl)-amid (2), (1S,3R)-2,2-Dimethyl-3-(2-methyl-propenyl)-cyclopropancarbonsäure-((1S,2S,5R)-2-isopropyl-5-methyl-cyclohexyl)-amid (3), (1S,3R)-2,2-Dimethyl-3-(2-methyl-propenyl)-cyclopropancarbonsäure-((1R,2R,5S)-2-isopropyl-6-methyl-cyclohexyl)-amid (4), (1S,3S)-2,2-Dimethyl-3-(2-methyl-propenyl)-cyclopropancarbonsäure-((1S,2S,5R)-2-isopropyl-5-methylcyclohexyl)-amid (5), (1S,3S)-2,2-Dimethyl-3-(2-methyl-propenyl)-cyclopropancarbonsäure-((1R,2R,5S)-2-isopropyl-5-methyl-cyclohexyl)-amid (6), (1R,3R)-2,2-Dimethyl-3-(2-methyl-propenyl)-cyclopropancarbonsäure-((1S,2S,5R)-2-isopropyl-5-methyl-cyclohexyl)-amid (7), (1R,3R)-2,2-Dimethyl-3-(2-methyl-propenyl)-cyclopropancarbonsäure-((1R,2R,5S)-2-isopropyl-5-methylcyclohexyl)-amid (8).

3. Verwendung nach Anspruch 1, wobei die Verbindung oder die Verbindungen der Formel (I) eine Verbindung oder Verbindungen der Formel (II) ist oder sind 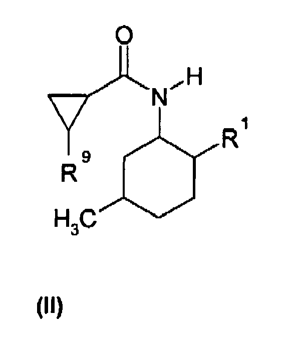 und wobei gilt:
R¹ bedeutet einen Alkylrest von 1 bis 3 C-Atomen;
R⁹ bedeutet Wasserstoff, einen Alkylrest mit 5 bis 12 C-Atomen oder einen Alkenylrest mit 5 bis 12 C-Atomen.

4. Verwendung nach Anspruch 3, wobei die Verbindung oder die Verbindungen der Formel (II) eine Verbindung ist oder Verbindungen sind, ausgewählt aus der Gruppe bestehend aus:
Cyclopropancarbonsäure((1R,2S,5R)-2-isopropyl-5-methyl-cyclohexyl)-amid (9),
Cyclopropancarbonsäure((1S,2R,5S)-2-isopropyl-5-methyl-cyclohexyl)-amid (10),
Cyclopropancarbonsäure((1S,2S,5R)-2isopropyl-5-methyl-cyclohexyl)-amid (11),
Cyclopropancarbonsäure((1R,2R,5S)-2-isopropyl-5-methyl-cyclohexyl)-amid (12),
Cyclopropancarbonsäure((1R,2R,5R)-2-isopropyl-5-methyl-cyclohexyl)-amid (13),
Cyclopropancarbonsäure((1S,2S,5S)-2-isopropyl-6-methyl-cyclohexyl)-amid (14),
Cyclopropancarbonsäure((1S,2R,5R)-2-isopropyl-5-methyl-cyclohexyl)-amid (15) und
Cyclopropancarbonsäure((1R,2S,5S)-2-isopropyl-5-methyl-cyclohexyl)-amid (16).

5. Verwendung einer Verbindung oder einer Mischung von Verbindungen der Formel (I) oder (II), wie in einem der vorangehenden Ansprüche definiert zum Erzeugen, Modifizieren oder Verstärken eines Umami-Geschmackes.

6. Zusammensetzung, insbesondere zum Verzehr geeignete Zusammensetzung, umfassend oder bestehend aus
- einer geschmacklich wirksamen Menge einer oder mehrerer Verbindungen der Formel (I) oder (II) wie in einem der Ansprüche 1 bis 4 definiert und außerdem
- einen oder mehrere zum Verzehr geeignete Bestandteile.

7. Zusammensetzung nach Anspruch 6, wobei die weiteren Bestandteile sind:
- feste Trägerstoffe oder
- feste Trägerstoffe und Aromakompositionen oder
- Wasser, eine Ölphase, ein oder mehrere W/O-Emulgatoren, gegebenenfalls ein oder mehrere Antioxidantien und gegebenenfalls ein oder mehrere Stoffe zur Verstärkung einer antioxidativen Wirkung.

8. Zusammensetzung nach Anspruch 7, wobei die weiteren Bestandteile feste Trägerstoffe sind und das Gewichtsverhältnis der Gesamtmasse der Verbindungen der Formel (I) oder (II), wie in einem der Ansprüche 1 bis 4 definiert zu den festen Trägerstoffen im Bereich von 1 : 10 bis 1 : 100000, bevorzugt im Bereich von 1 : 100 bis 1 : 20000, besonders bevorzugt im Bereich von 1 : 1000 bis 1 : 5000, liegt, bezogen auf die Trockenmasse der Zusammensetzung.

9. Zusammensetzung nach Anspruch 7, umfassend oder bestehend aus
- 0,01 bis 0,1 Gew.-%, einer oder mehrerer Verbindungen der Formel (I) oder (II) wie in einem der Ansprüche 1 bis 4 definiert,
- 5 bis 30 Gew.-% Wasser,
- 50 bis 90 Gew.-% einer Ölphase,
- 0,1 bis 5 Gew.-% eines verzehrbaren W/O-Emulgators sowie
- gegebenenfalls ein oder mehrere Antioxidantien und gegebenenfalls ein oder mehrere Stoffe zur Verstärkung einer antioxidativen Wirkung.

10. Der Ernährung, der Mundpflege oder dem Genuss dienende (i) gebrauchs- oder verzehrfertige Zubereitung oder (ii) Halbfertigware, umfassend
- eine geschmacklich wirksame Menge einer oder mehrerer Verbindungen der Formel (I) oder (II) wie in einem der Ansprüche 1 bis 4 definiert oder
- eine Zusammensetzung nach einem der Ansprüche 6 bis 9.

11. Der Ernährung, der Mundpflege oder dem Genuss dienende gebrauchs- oder verzehrfertige Zubereitung nach Anspruch 10, umfassend 0,01 ppm bis 100 ppm, bevorzugt 0,1 ppm bis 50 ppm, besonders bevorzugt 1 ppm bis 30 ppm an einer oder mehrerer der Verbindungen der Formel (I) oder (II) wie in einem der Ansprüche 1 bis 4 definiert, bezogen auf das Gesamtgewicht der verzehrfertigen Zubereitung.

12. Zusammensetzung, Zubereitung oder Halbfertigware nach einem der Ansprüche 6 bis 11, zusätzlich umfassend eine Substanz zum Maskieren oder Vermindern eines unangenehmen Geschmackseindrucks und/oder eine Substanz zum Verstärken des angenehmen Geschmackseindrucks eines angenehm schmeckenden Stoffes.

13. Verfahren zum Erzeugen, Modifizieren oder Verstärken eines Geschmacks in einer der Ernährung oder dem Genuss dienenden (i) verzehrfertigen Zubereitung oder (ii) Halbfertigware, mit folgendem Schritt:
- Vermischen einer geschmacklich wirksamen Menge einer oder mehrerer Verbindungen der Formel (I) oder (II) wie in einem der Ansprüche 1 bis 4 definiert oder einer Zusammensetzung nach einem der Ansprüche 6 bis 9 oder 12 mit einem oder mehreren weiteren Bestandteilen der (i) verzehrfertigen Zubereitung oder der (ii) Halbfertigware
und/oder
- Applizieren einer geschmacklich wirksamen Menge einer oder mehrerer Verbindungen der Formel (I) oder (II) wie in einem der Ansprüche 1 bis 4 definiert
oder einer Zusammensetzung nach einem der Ansprüche 6 bis 9 oder 12 auf einen oder mehrere weitere Bestandteile der (i) verzehrfertigen Zubereitung oder der (ii) Halbfertigware
und/oder
- Einbetten einer geschmacklich wirksamen Menge einer oder mehrerer Verbindungen der Formel (I) oder (II) wie in einem der Ansprüche 1 bis 4 definiert
oder einer Zusammensetzung nach einem der Ansprüche 6 bis 9 oder 12 in ein Hüll- oder Matrixmaterial.

14. Verfahren nach Anspruch 13, zum Erzeugen, Modifizieren oder Verstärken eines Umami-Geschmacks.

15. Verbindung der Formel (I) oder Mischung von Verbindungen der Formel (I) 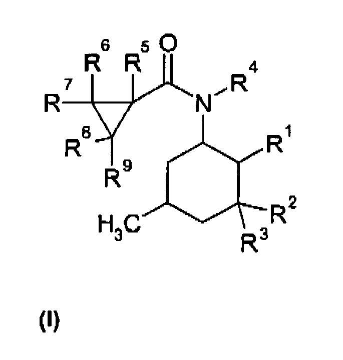 wobei gilt:
R¹, R², R³ bedeuten jeweils unabhängig voneinander Wasserstoff oder einen Alkylrest von 1 bis 3 C-Atomen, wobei mindestens ein Rest ungleich H ist;
R⁴ bedeutet Wasserstoff, einen Alkylrest von 1 bis 6 C-Atomen oder einen Alkenylrest von 2 bis 6 C-Atomen;
R⁵, R⁶, R⁷, R⁸ bedeuten jeweils unabhängig voneinander Wasserstoff oder Methyl;
R⁹ bedeutet Wasserstoff, einen Alkylrest mit 5 bis 12 C-Atomen oder einen Alkenylrest mit 5 bis 12 C-Atomen
mit der Maßgabe, dass die Verbindungen der Formel (I), die unter die Formel (III) 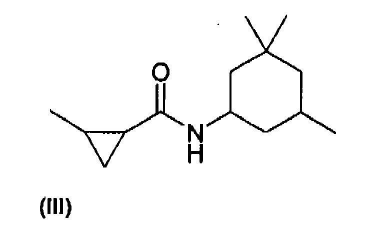 fallen, ausgenommen sind.

16. Verbindung nach Anspruch 15 oder Mischung von Verbindungen nach Anspruch 15, wobei die Verbindung oder die Verbindungen eine Verbindung ist oder Verbindungen sind, ausgewählt aus der Gruppe bestehend aus:
(1R,3S)-2,2-Dimethyl-3-(2-methyl-propenyl)-cyclopropancarbonsäure-((1S,25,5R)-2-isopropyl-5-methyl-cyclohexyl)-amid (1), (1R,3S)-2,2-Dimethyl-3-(2-methyl-propenyl)-cyclopropancarbonsäure-((1R,2R,5S)-2-isopropyl-5-methylcyclohexyl)-amid (2), (1S,3R)-2,2-Dimethyl-3-(2-methyl-propenyl)-cyclopropancarbonsäure-((1S,2S,5R)-2-isopropyl-5-methyl-Cyclohexyl)-amid (3), (1S,3R)-2,2-Dimethyl-3-(2-methyl-propenyl)-cyclopropancarbonsäure-((1R,2R,5S)-2-isopropyl-5-methyl-cyclohexyl)-amid (4), (1S,3S)-2,2-Dimethyl-3-(2-methyl-propenyl)-cyclopropancarbonsäure-((1S,2S,5R)-2-isopropyl-5-methylcyclohexyl)-amid (5), (1S,3S)-2,2-Dimethyl-3-(2-methyl-propenyl)-cyclopropancarbonsäure-((1R,2R,5S)-2-isopropyl-5-methyl-cyclohexyl)-amid (6), (1R,3R)-2,2-Dimethyl-3-(2-methyl-propenyl)-cyclopropancarbonsäure-((1S,2S,5R)-2-isopropyl-5-methyl-cyclohexyl)-amid (7), (1R,3R)-2,2-Dimethyl-3-(2-methyl-propenyl)-cyclopropancarbonsäure-((1R,2R,5S)-2-isopropyl-5-methylcyclohexyl)-amid (8).

17. Verbindung nach Anspruch 15 oder Mischung von Verbindungen nach Anspruch 15, wobei die Verbindung oder die Verbindungen der Formel (I) eine Verbindung oder Verbindungen der Formel (II) ist oder sind 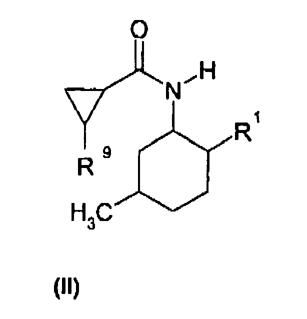 und wobei gilt:
R¹ bedeutet einen Alkylrest von 1 bis 3 C-Atomen;
R⁹ bedeutet Wasserstoff, einen Alkylrest mit 5 bis 12 C-Atomen oder einen Alkenylrest mit 5 bis 12 C-Atomen.

18. Verbindung nach Anspruch 17 oder Mischung von Verbindungen nach Anspruch 17, wobei die Verbindung oder die Verbindungen der Formel (II) eine Verbindung ist oder Verbindungen sind, ausgewählt aus der Gruppe bestehend aus:
Cyclopropancarbonsäure((1R,2S,5R)-2-isopropyl-5-methyl-cyclohexyl)-amid (9),
Cyclopropancarbonsäure((1S,2R,5S)-2-isopropyl-5-methyl-cyclohexyl)-amid (10),
Cyclopropancarbonsäure((1S,2S,5R)-2-isopropyl-5-methyl-cyclohexyl)-amid (11),
Cyclopropancarbonsäure((1R,2R,5S)-2-isopropyl-5-methyl-cyclohexyl)-amid (12),
Cyclopropancarbonsäure((1R,2R,5R)-2-isopropyl-5-methyl-cyclohexyl)-amid (13),
Cyclopropancarbonsäure((1S,2S,5S)-2-isopropyl-5-methyl-cyclohexyl)-amid (14),
Cyclopropancarbonsäure((1S,2R,5R)-2-isopropyl-5-methyl-cyclohexyl)-amid (15) und
Cyclopropancarbonsäure((1R,2S,5S)-2-isopropyl-5-methyl-cyclohexyl)-amid (16).

19. Verfahren zur Herstellung einer Verbindung der Formeln (11) und/oder (12) bzw. einer Mischung umfassend oder bestehend aus den Formeln (11) und/oder (12) wie definiert in Anspruch 18, umfassend die folgenden Schritte:
(a) Durchführen einer Leuckart-Wallach Reaktion ausgehend von enantiomerenreinen oder racemischen Menthon zu den entsprechenden Formamiden der Formel (III), 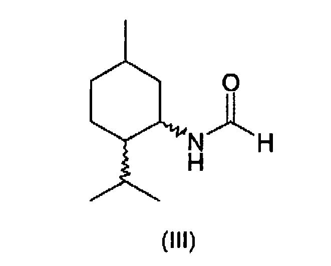
(b) Durchführen einer fraktionierten Kristallisation der Neo-Menthylformamide (Formeln (IV) und/oder (V)) 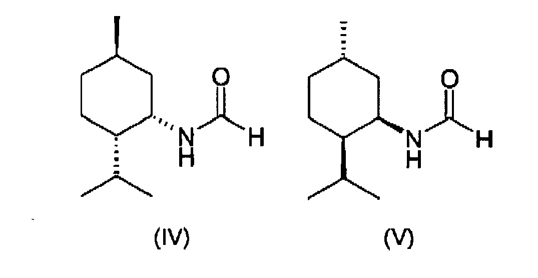
(c) Verseifung der Neo-Menthylformamide mit einer starken Säure zu (VI) und/oder (VII) und 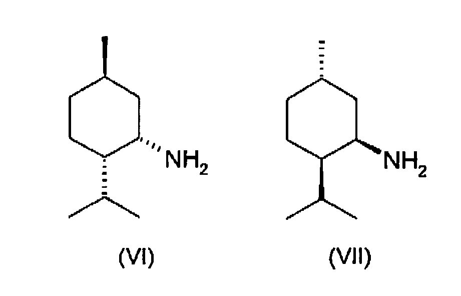
(d) Umsetzung des Amins entweder mit (i) Cyclopropancarbonsäurechlorid oder mit (ii) Cyclopropancarbonsäure zu (11) und/oder (12)

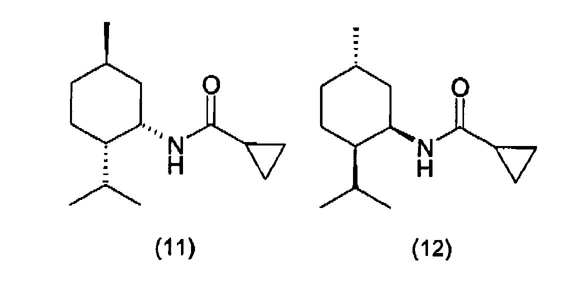

## Claims

1. Use of a compound of formula (I) or of a mixture of compounds of formula (I) 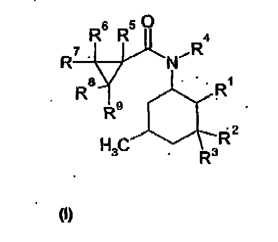 wherein:
R¹, R², R³ each independently of the others denotes hydrogen or an alkyl radical having from 1 to 3 carbon atoms, at least one radical being other than H;
R⁴ denotes hydrogen, an alkyl radical having from 1 to 6 carbon atoms or an alkenyl radical having from 2 to 6 carbon atoms;
R⁵, R⁶, R⁷, R⁸ each independently of the others denotes hydrogen or methyl;
R⁹ denotes hydrogen, an alkyl radical having from 5 to 12 carbon atoms or an alkenyl radical having from 5 to 12 carbon atoms,
as a flavouring or flavouring mixture.

2. Use according to claim 1, wherein the compound or the compounds of formula (I) is a compound or are compounds selected from the group consisting of:
(1R,3S)-2,2-dimethyl-3-(2-methyl-propenyl)-cyclopropanecarboxylic acid ((1S,2S,5R)-2-isopropyl-5-methyl-cyclohexyl)-amide (1), (1R,3S)-2,2-dimethyl-3-(2-methyl-propenyl)-cyclopropanecarboxylic acid ((1R,2R,5S)-2-isopropyl-5-methyl-cyclohexyl)-amide (2), (1S,3R)-2,2-dimethyl-3-(2-methyl-propenyl)-cyclopropanecarboxylic acid ((1S,2S,5R)-2-isopropyl-5-methyl-cyclohexyl)-amide (3), (1S,3R)-2,2-dimethyl-3-(2-methyl-propenyl)-cyclopropanecarboxylic acid ((1R,2R,5S)-2-isopropyl-5-methyl-cyclohexyl)-amide (4), (1S,3S)-2,2-dimethyl-3-(2-methyl-propenyl)-cyclopropanecarboxylic acid ((1S,2S,5R)-2-isopropyl-5-methyl-cyclohexyl)-amide (5), (1S,3S)-2,2-dimethyl-3-(2-methyl-propenyl)-cyclopropanecarboxylic acid ((1R,2R,5S)-2-isopropyl-5-methyl-cyclohexyl)-amide (6), (1R,3R)-2,2-dimethyl-3-(2-methyl-propenyl)-cyclopropanecarboxylic acid ((1S,2S,5R)-2-isopropyl-5-methyl-cyclohexyl)-amide (7), (1R,3R)-2,2-dimethyl-3-(2-methyl-propenyl)-cyclopropanecarboxylic acid ((1R,2R,5S)-2-isopropyl-5-methyl-cyclohexyl)-amide (8).

3. Use according to claim 1, wherein the compound or the compounds of formula (I) is or are a compound or compounds of formula (II) 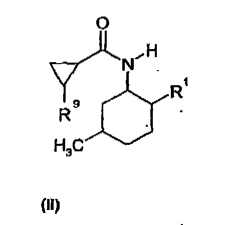 wherein:
R¹ denotes an alkyl radical having from 1 to 3 carbon atoms;
R⁹ denotes hydrogen, an alkyl radical having from 5 to 12 carbon atoms or an alkenyl radical having from 5 to 12 carbon atoms.

4. Use according to claim 3, wherein the compound or the compounds of formula (II) is a compound or are compounds selected from the group consisting of:
cyclopropanecarboxylic acid ((1R,2S,5R)-2-isopropyl-5-methyl-cyclohexyl)-amide (9),
cyclopropanecarboxylic acid ((1S,2R,5S)-2-isopropyl-5-methyl-cyclohexyl)-amide (10),
cyclopropanecarboxylic acid ((1S,2S,5R)-2-isopropyl-5-methyl-cyclohexyl)-amide (11),
cyclopropanecarboxylic acid ((1R,2R,5S)-2-isopropyl-5-methyl-cyclohexyl)-amide (12),
cyclopropanecarboxylic acid ((1R,2R,5R)-2-isopropyl-5-methyl-cyclohexyl)-amide (13),
cyclopropanecarboxylic acid ((1S,2S,5S)-2-isopropyl-5-methyl-cyclohexyl)-amide (14),
cyclopropanecarboxylic acid ((1S,2R,5R)-2-isopropyl-5-methyl-cyclohexyl)-amide (15) and
cyclopropanecarboxylic acid ((1R,2S,5S)-2-isopropyl-5-methyl-cyclohexyl)-amide (16).

5. Use of a compound or of a mixture of compounds of formula (I) or (II) as defined in any one of the preceding claims to produce, modify or enhance an umami taste.

6. Composition, in particular composition suitable for consumption, comprising or consisting of
- an amount, effective in terms of taste, of one or more compounds of formula (I) or (II) as defined in any one of claims 1 to 4 and in addition
- one or more constituents suitable for consumption.

7. Composition according to claim 6, wherein the further constituents are:
- solid carriers or
- solid carriers and flavour compositions or
- water, an oil phase, one or more W/O emulsifiers, optionally one or more antioxidants and optionally one or more substances for enhancing an antioxidant effect.

8. Composition according to claim 7, wherein the further constituents are solid carriers and the weight ratio of the total weight of the compounds of formula (I) or (II) as defined in any one of claims 1 to 4 to the solid carriers is in the range from 1 : 10 to 1 : 100,000, preferably in the range from 1 : 100 to 1 : 20,000, particularly preferably in the range from 1 : 1000 to 1 : 5000, based on the dry weight of the composition.

9. Composition according to claim 7, comprising or consisting of
- from 0.01 to 0.1 wt.% of one or more compounds of formula (I) or (II) as defined in any one of claims 1 to 4,
- from 5 to 30 wt.% water,
- from 50 to 90 wt.% of an oil phase,
- from 0.1 to 5 wt.% of an edible W/O emulsifier and
- optionally one or more antioxidants and optionally one or more substances for enhancing an antioxidant effect.

10. Ready-to-use or ready-to-eat preparation (i) or semi-finished product (ii) for nutrition, oral care or enjoyment, comprising
- an amount, effective in terms of taste, of one or more compounds of formula (I) or (II) as defined in any one of claims 1 to 4 or
- a composition according to any one of claims 6 to 9.

11. Ready-to-use or ready-to-eat preparation for nutrition, oral care or enjoyment according to claim 10, comprising from 0.01 ppm to 100 ppm, preferably from 0.1 ppm to 50 ppm, particularly preferably from 1 ppm to 30 ppm, of one or more of the compounds of formula (I) or (II) as defined in any one of claims 1 to 4, based on the total weight of the ready-to-eat preparation.

12. Composition, preparation or semi-finished product according to any one of claims 6 to 11, additionally comprising a substance for masking or reducing an unpleasant taste impression and/or a substance for enhancing the pleasant taste impression of a substance having a pleasant taste.

13. Process for producing, modifying or enhancing a taste in (i) a ready-to-eat preparation or (ii) a semi-finished product for nutrition or enjoyment, comprising the following step:
- mixing an amount, effective in terms of taste, of one or more compounds of formula (I) or (II) as defined in any one of claims 1 to 4 or of a composition according to any one of claims 6 to 9 or 12 with one or more further constituents of (i) the ready-to-eat preparation or (ii) the semi-finished product
and/or
- applying an amount, effective in terms of taste, of one or more compounds of formula (I) or (II) as defined in any one of claims 1 to 4
or of a composition according to any one of claims 6 to 9 or 12
to one or more further constituents of (i) the ready-to-eat preparation or (ii) the semi-finished product
and/or
- embedding an amount, effective in terms of taste, of one or more compounds of formula (I) or (II) as defined in any one of claims 1 to 4
or of a composition according to any one of claims 6 to 9 or 12
in a coating or matrix material.

14. Process according to claim 13 for producing, modifying or enhancing an umami taste.

15. Compound of formula (I) or mixture of compounds of formula (I) 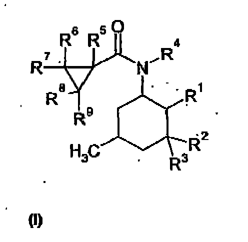 wherein:
R¹, R², R³ each independently of the others denotes hydrogen or an alkyl radical having from 1 to 3 carbon atoms, at least one radical being other than H;
R⁴ denotes hydrogen, an alkyl radical having from 1 to 6 carbon atoms or an alkenyl radical having from 2 to 6 carbon atoms;
R⁵, R⁶, R⁷, R⁸ each independently of the others denotes hydrogen or methyl;
R⁹ denotes hydrogen, an alkyl radical having from 5 to 12 carbon atoms or an alkenyl radical having from 5 to 12 carbon atoms,
with the proviso that the compounds of formula (I) that fall under formula (III) 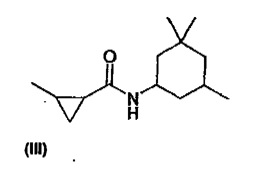 are excluded.

16. Compound according to claim 15 or mixture of compounds according to claim 15, wherein the compound or the compounds is a compound or are compounds selected from the group consisting of:
(1R,3S)-2,2-dimethyl-3-(2-methyl-propenyl)-cyclopropanecarboxylic acid ((1S,2S,5R)-2-isopropyl-5-methyl-cyclohexyl)-amide (1), (1R,3S)-2,2-dimethyl-3-(2-methyl-propenyl)-cyclopropanecarboxylic acid ((1R,2R,5S)-2-isopropyl-5-methyl-cyclohexyl)-amide (2), (1S,3R)-2,2-dimethyl-3-(2-methyl-propenyl)-cyclopropanecarboxylic acid ((1S,2S,5R)-2-isopropyl-5-methyl-cyclohexyl)-amide (3), (1S,3R)-2,2-dimethyl-3-(2-methyl-propenyl)-cyclopropanecarboxylic acid ((1R,2R,5S)-2-isopropyl-5-methyl-cyclohexyl)-amide (4), (1S,3S)-2,2-dimethyl-3-(2-methyl-propenyl)-cyclopropanecarboxylic acid ((1S,2S,5R)-2-isopropyl-5-methyl-cyclohexyl)-amide (5), (1S,3S)-2,2-dimethyl-3-(2-methyl-propenyl)-cyclopropanecarboxylic acid ((1R,2R,5S)-2-isopropyl-5-methyl-cyclohexyl)-amide (6), (1R,3R)-2,2-dimethyl-3-(2-methyl-propenyl)-cyclopropanecarboxylic acid ((1S,2S,5R)-2-isopropyl-5-methyl-cyclohexyl)-amide (7), (1R,3R)-2,2-dimethyl-3-(2-methyl-propenyl)-cyclopropanecarboxylic acid ((1R,2R,5S)-2-isopropyl-5-methyl-cyclohexyl)-amide (8).

17. Compound according to claim 15 or mixture of compounds according to claim 15, wherein the compound or the compounds of formula (I) is or are a compound or compounds of formula (II) 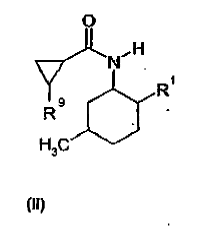 wherein:
R¹ denotes an alkyl radical having from 1 to 3 carbon atoms;
R⁹ denotes hydrogen, an alkyl radical having from 5 to 12 carbon atoms or an alkenyl radical having from 5 to 12 carbon atoms.

18. Compound according to claim 17 or mixture of compounds according to claim 17, wherein the compound or the compounds of formula (II) is a compound or are compounds selected from the group consisting of:
cyclopropanecarboxylic acid ((1R,2S,5R)-2-isopropyl-5-methyl-cyclohexyl)-amide (9),
cyclopropanecarboxylic acid ((1S,2R,5S)-2-isopropyl-5-methyl-cyclohexyl)-amide (10),
cyclopropanecarboxylic acid ((1S,2S,5R)-2-isopropyl-5-methyl-cyclohexyl)-amide (11),
cyclopropanecarboxylic acid ((1R,2R,5S)-2-isopropyl-5-methyl-cyclohexyl)-amide (12),
cyclopropanecarboxylic acid ((1R,2R,5R)-2-isopropyl-5-methyl-cyclohexyl)-amide (13),
cyclopropanecarboxylic acid ((1S,2S,5S)-2-isopropyl-5-methyl-cyclohexyl)-amide (14),
cyclopropanecarboxylic acid ((1S,2R,5R)-2-isopropyl-5-methyl-cyclohexyl)-amide (15) and
cyclopropanecarboxylic acid ((1R,2S,5S)-2-isopropyl-5-methyl-cyclohexyl)-amide (16).

19. Process for the preparation of a compound of formula (11) and/or (12) or of a mixture comprising or consisting of formula (11) and/or (12) as defined in claim 18, comprising the following steps:
(a) carrying out a Leuckart-Wallach reaction starting from enantiomerically pure or racemic menthone to give the corresponding formamides of formula (III) 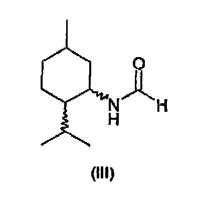
(b) carrying out fractional crystallisation of the neo-menthyl formamides (formula (IV) and/or (V)) 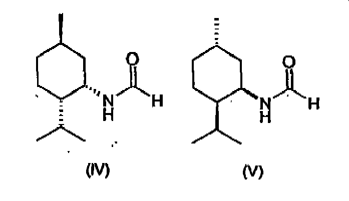
(c) saponification of the neo-menthyl formamides with a strong acid to give (VI) and/or (VII) and 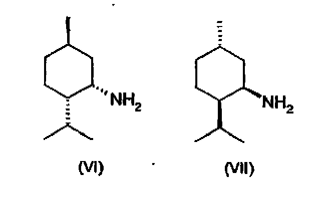
(d) reaction of the amine either with (i) cyclopropanecarboxylic acid chloride or with (ii) cyclopropanecarboxylic acid to give (11) and/or (12)

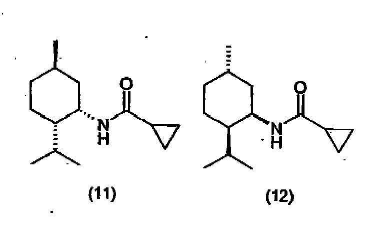

## Revendications

1. Utilisation d'un composé de la formule (I) ou d'un mélange de composés de la formule (I) 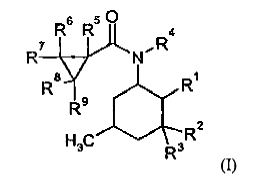 dans laquelle on a :
R¹, R², R³ signifient chacun, indépendamment l'un de l'autre, un hydrogène ou un radical alkyle avec 1 à 3 atomes de carbone, sachant qu'au moins un radical est différent de H;
R⁴ signifie un hydrogène, un radical alkyle avec 1 à 6 atomes de carbone ou un radical alcényle avec de 2 à 6 atomes de carbone;
R⁵, R⁶, R⁷, R⁸ signifient respectivement indépendamment l'un de l'autre un hydrogène ou un méthyle;
R⁹ signifie un hydrogène, un radical alkyle avec 5 à 12 atomes de carbone ou un radical alcényle avec 5 à 12 atomes de carbone
comme aromate ou comme mélange d'aromates.

2. Utilisation selon la revendication 1, sachant que le composé ou les composés de la formule (I) est un composé ou sont des composés choisis parmi le groupe consistant en :
le (1R,3S)-2,2-diméthyl-3-(2-méthyl-propényl)-(acide cyclopropane carboxylique)-((1S,2S,5R)-2-isopropyl-5-méthylcyclohexyl)-amide (1), le (1R,3S)-2,2-diméthyl-3-(2-méthyl-propényl)-(acide cyclopropane carboxylique)-((1R,2R,5S)-2-isopropyl-5-méthylcyclohexyl)-amide (2), le (1S,3R)-2,2-diméthyl-3-(2-méthyl-propényl)-(acide cyclopropane carboxylique)-((1S,2S,5R)-2-isopropyl-5-méthyl-cyclohexyl)-amide (3), le (1S,3R)-2,2-diméthyl-3-(2-méthyl-propényl)-(acide cyclopropane carboxylique)-((1R,2R,5S)-2-isopropyl-5-méthyl-cyclohexyl)-amide (4), le (1S,3S)-2,2-diméthyl-3-(2-méthyl-propényl)-(acide cyclopropane carboxylique)-((1S,2S,5R)-2-isopropyl-5-méthylcyclohexyl)-amide (5), le (1S,3S)-2,2-diméthyl-3-(2-méthyl-propényl)-(acide cyclopropane carboxylique)-((1R,2R,5S)-2-isopropyl-5-méthyl-cyclohexyl)-amide (6), le (1R,3R)-2,2-diméthyl-3-(2-méthyl-propényl)-(acide cyclopropane carboxylique)-((1S,2S,5R)-2-isopropyl-5-méthyl-cyclohexyl)-amide (7), le (1R,3R)-2,2-diméthyl-3-(2-méthyl-propényl)-(acide cyclopropane carboxylique)-((1R,2R,5S)-2-isopropyl-5-méthylcyclohexyl)-amide (8).

3. Utilisation selon la revendication 1, sachant que le composé ou les composés de la formule (I) est un composé ou sont des composés de la formule (II) 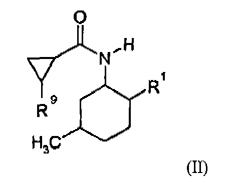 et dans laquelle on a :
R¹ signifie un radical alkyle avec 1 à 3 atomes de carbone;
R⁹ signifie un hydrogène, un radical alkyle avec 5 à 12 atomes de carbone ou un radical alcényle avec 5 à 12 atomes de carbone.

4. Utilisation selon la revendication 3, sachant que le composé ou les composés de la formule (II) est un composé ou sont des composés choisis parmi le groupe consistant en :
le ((1R,2S,5R)-2-isopropyl-5-méthyle-cyclohexyle)-amide de l'acide cyclopropane carboxylique (9),
le ((1S,2R,5S)-2-isopropyl-5-méthyle-cyclohexyle)-amide de l'acide cyclopropane carboxylique (10),
le ((1S,2S,5R)-2-isopropyl-5-méthyl-cyclohexyle)-amide de l'acide cyclopropane carboxylique (11),
le ((1R,2R,5S)-2-isopropyl-5-méthyl-cyclohexyle)-amide de l'acide cyclopropane carboxylique (12),
le ((1R,2R,5R)-2-isopropyl-S-méthyl-cyclohexyle)-amide de l'acide cyclopropane carboxylique (13),
le ((1S,2S,5S)-2-isopropyl-5-méthyl-cyclohexyle)-amide de l'acide cyclopropane carboxylique (14),
le ((1S,2R,5R)-2-isopropyl-5-méthyl-cyclohexyle)-amide de l'acide cyclopropane carboxylique (15)
et
le ((1R,2S,5S)-2-isopropyl-5-méthyl-cyclohexyle)-amide de l'acide cyclopropane carboxylique (16).

5. Utilisation d'un composé ou d'un mélange de composés de la formule (I) ou (II), comme définis dans une quelconque des revendications précédentes pour la production, la modification ou le renforcement d'une saveur umami.

6. Composition, en particulier composition appropriée à la consommation, comprenant ou consistant en
- une quantité gustativement efficace d'un ou de plusieurs composés de la formule (I) ou (II) comme définis dans une quelconque des revendications 1 à 4 et en outre
- en un ou plusieurs composants appropriés à la consommation.

7. Composition selon la revendication 6, sachant que les autres composants sont :
- des substances supports solides ou
- des substances supports solides et des compositions d'arômes ou
- de l'eau, une phase huileuse, un ou plusieurs émulsionnants eau-huile, le cas échéant un ou plusieurs antioxydants et le cas échéant un ou plusieurs produits pour le renforcement d'un effet antioxydant.

8. Composition selon la revendication 7, sachant que les autres composants sont des substances supports solides et que le rapport pondéral de la masse totale des composés de la formule (I) ou (II), comme définis dans l'une quelconque des revendications 1 à 4, aux substances supports solides, est compris dans l'intervalle de 1 : 10 à 1 : 100000, de préférence dans l'intervalle de 1 : 100 à 1 : 20000, de façon particulièrement préférée dans l'intervalle de 1 : 1000 à 1 : 5000, rapporté à la masse de produit sec de la composition.

9. Composition selon la revendication 7, comprenant ou consistant en
- 0,01 à 0,1 % en poids d'un ou de plusieurs composés de la formule (I) ou (II) comme définis dans l'une quelconque des revendications 1 à 4,
- 5 à 30 % en poids d'eau,
- 50 à 90 % en poids d'une phase huileuse,
- 0,1 à 5 % en poids d'un émulsionnant eau/huile consommable ainsi que
- le cas échéant un ou plusieurs antioxydants et le cas échéant un ou plusieurs produits pour le renforcement d'un effet antioxydant.

10. (i) Préparation ou (ii) produit semi-fini prêt à l'emploi ou prêt à la consommation servant à l'alimentation, aux soins de la bouche ou à la stimulation, comprenant
- une quantité gustativement efficace d'un ou de plusieurs composés de la formule (I) ou (II) comme définis dans l'une quelconque des revendications 1 à 4 ou
- une composition selon l'une quelconque des revendications 6 à 9.

11. Préparation prête à l'emploi ou prête à la consommation selon la revendication 10, servant à l'alimentation, aux soins de la bouche ou à la stimulation, comprenant, rapportés au poids total de la préparation prête à la consommation, 0,01 ppm à 100 ppm, de préférence 0,1 ppm à 50 ppm, de façon particulièrement préférée 1 ppm à 30 ppm d'un ou de plusieurs des composés de la formule (I) ou (II) comme définis dans l'une quelconque des revendications 1 à 4.

12. Composition, préparation ou produit semi-fini selon l'une quelconque des revendications 6 à 11, comprenant en plus une substance pour masquer ou diminuer une impression gustative désagréable et/ou une substance pour l'augmentation de l'impression gustative agréable d'un produit d'une saveur agréable.

13. Procédé pour la production, la modification ou le renforcement d'une saveur dans (i) une préparation prête à la consommation ou (ii) un produit semi-fini servant à l'alimentation ou à la stimulation, avec l'étape suivante :
- mélangeage d'une quantité gustativement efficace d'un ou de plusieurs composés des formules (I) ou (II) comme définis dans l'une quelconque des revendications 1 à 4 ou d'une composition selon l'une quelconque des revendications 6 à 9 ou 12 avec un ou plusieurs autres composants de la (i) préparation prête à la consommation ou du (ii) produit semi-fini
et/ou
- application d'une quantité gustativement efficace d'un ou de plusieurs composés des formules (I) ou (II) comme définis dans l'une quelconque des revendications 1 à 4
ou d'une composition selon l'une quelconque des revendications 6 à 9 ou 12 sur un ou plusieurs autres composants de la (i) préparation prête à la consommation ou du (ii) produit semi-fini
et/ou
- incorporation d'une quantité gustativement efficace d'un ou de plusieurs composés des formules (I) ou (II) comme définis dans l'une quelconque des revendications 1 à 4
ou d'une composition selon l'une quelconque des revendications 6 à 9 ou 12 dans un matériau enveloppe ou dans un matériau matrice.

14. Procédé selon la revendication 13, pour la production, la modification ou le renforcement d'une saveur umami.

15. Composé de la formule (I) ou mélange de composés de la formule (I) 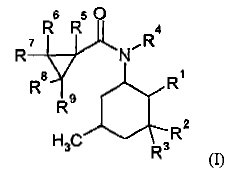 dans lequel on a :
R¹, R², R³ signifient respectivement indépendamment l'un de l'autre un hydrogène ou un radical alkyle de 1 à 3 atomes de carbone, sachant qu'au moins un radical est différent de H ;
R⁴ signifie un hydrogène, un radical alkyle de 1 à 6 atomes de carbone ou un radical alcényle de 2 à 6 atomes de carbone ;
R⁵, R⁶, R⁷, R⁸ signifient respectivement indépendamment l'un de l'autre un hydrogène ou un méthyle ;
R⁹ signifie un hydrogène, un radical alkyle avec 5 à 12 atomes de carbone ou un radical alcényle avec 5 à 12 atomes de carbone,
avec la spécification que les composés de la formule (I), qui tombent sous la formule (III) 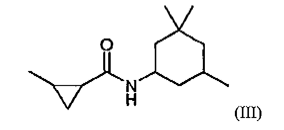 sont exclus.

16. Composé selon la revendication 15 ou mélange de composés selon la revendication 15, sachant que le composé ou les composés est un composé ou sont des composés choisis parmi le groupe consistant en :
le (1R,3S)-2,2-diméthyle-3-(2-méthyl-propényl)-(acide cyclopropane carboxylique)-((1S,2S,5R)-2-isopropyl-5-méthyl-cyclohexyl)-amide (1), le (1R,3S)-2,2-diméthyle-3-(2-méthyl-propényl)-(acide cyclopropane carboxylique)-((1R,2R,5S)-2-isopropyl-5-méthylcyclohexyl)-amide (2), le (1S,3R)-2,2-diméthyle-3-(2-méthyl-propényl)-(acide cyclopropane carboxylique)-((1S,2S,5R)-2-isopropyl-5-méthyl-cyclohexyl)-amide (3), le (1S,3R)-2,2-diméthyle-3-(2-méthyl-propényl)-(acide cyclopropane carboxylique)-((1R,2R,5S)-2-isopropyl-5-méthyl-cyclohexyl)-amide (4), le (1S,3S)-2,2-diméthyle-3-(2-méthyl-propényl)-(acide cyclopropane carboxylique)-((1S,2S,SR)-2-isopropyl-5-méthylcyclohexyl)-amide (5), le (1S,3S)-2,2-diméthyle-3-(2-méthyl-propényl)-(acide cyclopropane carboxylique)-((1R,2R,5S)-2-isopropyl-5-méthyl-cyclohexyl)-amide (6), le (1R,3R)-2,2-diméthyle-3-(2-méthyl-propényl)-(acide cyclopropane carboxylique)-((1S,2S,5R)-2-isopropyl-5-méthyl-cyclohexyl)-amide (7), le (1R,3R)-2,2-diméthyle-3-(2-méthyl-propényl)-(acide cyclopropane carboxylique)-((1R,2R,5S)-2-isopropyl-5-méthylcyclohexyl)-amide (8).

17. Composé selon la revendication 15 ou mélange de composés selon la revendication 15, sachant que le composé ou les composés de la formule (I) est ou sont un composé ou des composés de la formule (II) 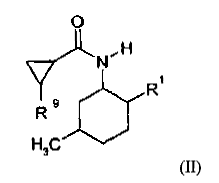 et dans laquelle on a :
R¹ signifie un radical alkyle de 1 à 3 atomes de carbone;
R⁹ signifie un hydrogène, un radical alkyle avec 5 à 12 atomes de carbone ou un radical alcényle avec 5 à 12 atomes de carbone.

18. Composé selon la revendication 17 ou mélange de composés selon la revendication 17, sachant que le composé ou les composés de la formule (II) est un composé ou sont des composés choisis parmi le groupe consistant en :
le ((1R,2S,5R)-2-isopropyl-5-méthyle-cyclohexyle)-amide de l'acide cyclopropane carboxylique (9),
le ((1S,2R,5S)-2-isopropyl-5-méthyle-cyclohexyle)-amide de l'acide cyclopropane carboxylique (10),
le ((1S,2S,5R)-2-isopropyl-5-méthyl-cyclohexyle)-amide de l'acide cyclopropane carboxylique (11),
le ((1R,2R,5S)-2-isopropyl-5-méthyl-cyclohexyle)-amide de l'acide cyclopropane carboxylique (12),
le ((1R,2R,5R)-2-isopropyl-5-méthyl-cyclohexyle)-amide de l'acide cyclopropane carboxylique (13),
le ((1S,2S,5S)-2-isopropyl-5-méthyl-cyclohexyle)-amide de l'acide cyclopropane carboxylique (14),
le ((15,2R,5R)-2-isopropyl-5-méthyl-cyclohexyle)-amide de l'acide cyclopropane carboxylique (15)
et
le ((1R,2S,5S)-2-isopropyl-5-méthyl-cyclohexyle)-amide de l'acide cyclopropane carboxylique (16).

19. Procédé pour la préparation d'un composé des formules (11) et/ou (12), respectivement d'un mélange comprenant ou consistant en les formules (11) et/ou (12) comme définies dans la revendication 18, comprenant les étapes suivantes :
(a) exécution d'une réaction de Leuckart-Wallach en partant de menthone énantiomère pure ou racémique pour donner les formamides correspondants de la formule (III), 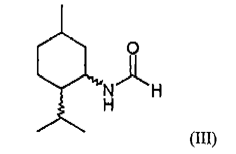
(b) exécution d'une cristallisation fractionné du néo-menthylformamide (formules (IV) et/ou (V)) 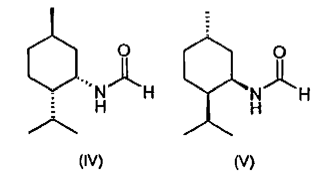
(c) saponification du néo-menthylformamide avec un acide fort pour donner (VI) et/ou (VII) et 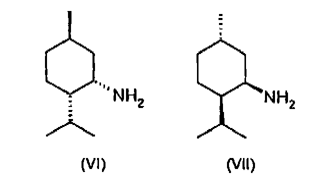
(d) conversion de l'amine soit avec du (i) chlorure d'acide cyclopropane carboxylique, soit avec de (ii) l'acide cyclopropane carboxylique pour donner (11) et/ou (12)
